# EUROPEAN PATENT APPLICATION

(11) **EP 1 439 233 A1**
(43) Date of publication of application: **21.07.2004**
(21) Application number: 03290116.7
(22) Date of filing: 16.01.2003
(51) Int. Cl.: C12N 15/82, C12N 15/29, C12N 5/14, A01H 5/00, C12Q 1/68

(54) **Polypeptides involved in floral development and genes encoding the same**

(71) Applicant: Genoplante-Valor, 91025 Evry Cedex (FR)
(72) Inventor: Foucher, Fabrice, 49100 Angers (FR); Rameau, Catherine, 75014 Paris (FR); Lejeune-Henaut, Isabelle, 80240 Roisel (FR)
(74) Representative: Vialle-Presles, Marie José

(57) **Abstract**

The present invention relates to the identification of polypeptides active in floral transition and/or floral development and to their use for regulating one of this floral development step.

## Description

The present invention relates to means and methods for modulating the floral development in plants.

The floral transition is the switch from vegetative growth to reproductive growth: flower meristem is produced from the apical meristem. This switch is controlled by physiological signals, and by a complex genetic network that integrate environmental (photoperiod and temperature) and endogenous (stage of the plant) conditions.

The control of floral transition is of great importance for agriculture and breeding. For instance, in the case of plants cultivated for their flowers or fruits, advancing the flowering can shorten the production time while retarding it can reduce the risk of damages due to harsh climatic conditions such as frost. In the case of plants cultivated for their roots or leaves, delaying the reproductive growth can favor the vegetative growth, resulting in a higher yield.

This interest of controlling the switch from vegetative to reproductive stage, in particular in order to influence the floral development, is illustrated for instance by the following Patents and Patent applications:
The International Application WO 98/54328 provides compositions and methods for affecting the transition from vegetative to reproductive growth by providing new genes called OsMADs6-8 which, when expressed in plants results in such phenotypes as apical dominance or dwarfism and early flowering. The invention disclosed in WO 98/54328 is directed specifically to MADS genes of *Oryza sativa.*
The United States Patent 6,265,637 discloses a genetic control of flowering in plants and the cloning and expression of genes involved therein and more particulary the isolation, cloning and expression of the Late Elongated Hypocotyl (LHY) gene of *Arabidopsis thaliana.*
The US Patent 5,744,693 reports the use of recombinant nucleic acids derived from an agamous gene to produce plants having a phenotype characterized by altered floral development.

Within the plant kingdom a number of distinct flowers and inflorescences can be found. There are two basic types of inflorescence: indeterminate (where the apical meristem grows indefinitely and flowers arise from the axillary meristem) and determinate (where apical meristem is converted into terminal flower). Examples of indeterminate inflorescences are those of *Arabidopsis thaliana,* snapdragon (*Antirrhinum majus*) or garden pea (*Pisum sativum*) are also designated as raceme, and example of determinate inflorescences are those of tobacco or tomato.

Mutants presenting modifications in floral development and architecture have been characterized in different plants (*Arabidopsis* (Blasquez, 2000), snapdragon, pea (Reid, 1996 ; Weller, 1997) or petunia. These genes are well conserved and play similar roles.

For instance *LEAFY* (*LFY*), which was isolated in *Arabidopsis,* is a key gene involved in floral development (Weigel, 1992). Orthologues of *LFY* were found in other plants *: FLORICAULA* in snapdragon (Coen, 1990), *UNIFOLIATA* in pea (Hofer, 1997), *ALF* in petunia (Souer, 1998). Nevertheless different regulation processes or new roles were found in certain circumstances. For example, *UNI* is involved not only in flower development but also in compound leaf development, a new function not described for LFY or FLO (Hofer, 1997).

*CENTRORADIALIS* is involved in inflorescence architecture in snapdragon (Bradley, 1996). The *cen* mutation leads to the conversion of the indeterminate inflorescence into a terminal flower. *CEN* is induced after *FLO* and requires *FLO* for its expression. *CEN* may prevent the conversion of the apical meristem to flower meristem by repressing floral meristem genes such as *FLO* (Bradley, 1996). Orthologues of *CEN* were found in different species: *Terminal Flower 1*(*TFL1,* Ohshima, 1997 ; Bradley, 1997) in *Arabidopsis, SELF PRUNING* (*SP,* Pnueli, 1998) in tomato, *CET* in tobacco (Amaya, 1999) or *LpTFL1* in *Lolium* perenne (Jensen, 2001).

In *Arabidopsis, tfl1* mutants present a phenotype similar to cen mutants: indeterminate growth is converted into determinate (Bradley, 1997). However *tfl1* mutants flower earlier than the wild type. The early flowering was not observed in snapdragon. *TFL1* may play a role in inflorescence meristem identity but also in floral initiation control. It was proposed that these two distinct roles are in fact only one, with *TFL1* controlling the length of both the vegetative and reproductive phases (Ratcliffe, 1998). In determinate tomato, sp mutants present a reduction of the number of sympodial units and termination of the growth by two successive inflorescences (Pnueli, 1998). No difference in flowering time was observed in sp mutants.

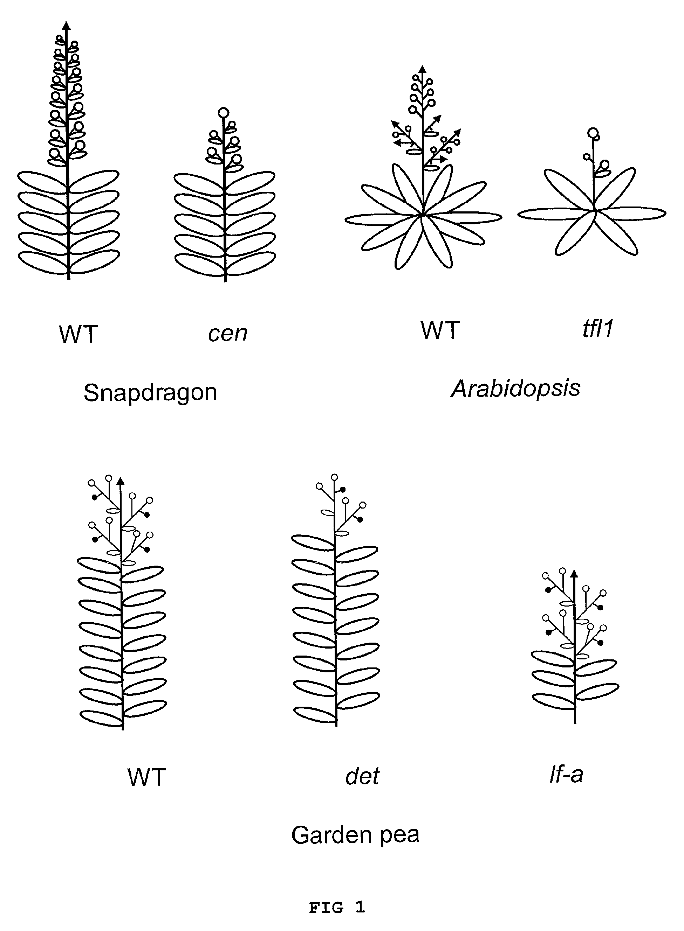

A schematic representation of different architecture of plants and their *TFL1* homologue mutants is shown in Figure 1.

*CEN, TFL1* and SP have some similarity to a family of mammalian phosphatidylethanolamine-binding proteins (PBPs). Recent crystallography analysis reveals that CEN may be involved in kinase interaction (Banfield, 2000). In tomato, SP was shown to interact with multiple proteins and was proposed to be a modular protein with the potential to interact with a variety of signaling proteins (Pnueli, 2001). Expression analysis revealed that these genes are mainly expressed in the shoot apical meristem in the region below the terminal meristem. *CEN* is induced during floral initiation (Bradley, 1996), whereas *TFL1* expression is also found during vegetative phase (expression that could explain the role of *TFL1* in delaying flowering in *Arabidopsis* (Bradley, 1997)). In tomato, *SP* expression is found in all meristems at the vegetative or floral stage; no significant variations are observed (Pnueli, 1998).

Analysis of mutants and sequencing of the whole *Arabidopsis* genome reveal that *TFL1* genes belong to a small family of genes (at least 6 genes) with functional divergence (Mimida, 2001). One of them (*FT* for FLOWERING LOCUS T) has an antagonist role of *TFL1* by promoting flowering in *Arabidopsis* (Kardailsky, 1999; Kobayashi, 1999). An *atc* mutant (*Arabidopsis thaliana CENTRORADIALIS* homologue) shows no flowering phenotype but overexpression of the gene can complement *TFL1* mutants (Mimida, 2001).

Pea is an indeterminate flowering plant such as *Arabidopsis* and snapdragon. The apical meristem grows indefinitely, and two flowers arise from axillary meristems in the leaf axils. During senescence the apical meristem ceases growing and is converted into a stub (terminal meristem with epidermal hairs)

The floral initiation and development in pea has been studied for many decades. Based on physiological, mutational analysis, a model for this system, which involves both a floral inhibitor and a stimulus, has been developed (Reid, 1996). The integration of the signals takes place in the apex and is controlled by the *Lf* (*Late Flowering*) locus. Four natural alleles of *Lf* are known: *Lf-d, Lf, lf*, *lf*-*a*: in maximal inductive conditions the minimum node of flower initiation is of respectively 15 for *Lf-d,* 11 for *Lf*, 8 for *lf* and 5 for *lf*-*a*. Physiological and graft experiments show that *Lf* is active in the shoot and the different alleles determinate threshold sensitivity of the apical meristem to flowering signals. Mutation of *Lf* leads to plants presenting an early flowering phenotype. *LF* may be a repressor of flowering as *TFL1* in *Arabidopsis.*

A pea mutant, *det* (for determinate) presents a phenotype which is similar to *tfl1* or *cen* mutants (Singer et al., 1991; Swiecicki, 1987). In *det* mutants, few flowers are produced and a terminal flower arises. Scanning electron microscopy analysis shows that the terminal flower is in fact arising from axillary meristem while the terminal meristem is converted into a stub (Singer et al., 1991). A real terminal flower can be obtained in pea by crossing *det* mutant with another mutant, *veg1* (Singer, 1999). *Veg1* (for Vegetative1) is a pea mutant, which remains vegetative, and can flower only under certain circumstances (Reid, 1984). The *det veg1* double mutant replaces the terminal stub with a flower (Singer, 1999).

However, the complex genetic pattern underlying the floral transition remains poorly understood, and there is still a need to identify genes involved in the modulation of floral development and/or the maintenance of the apical meristem.

The inventors have now found that the *determinate* and *Late flowering* phenotypes in pea were separately controlled by two *TFL1* homologues.

The first gene characterized by the inventors, hereinafter designated *PsTFL1a*, segregated with the *det* mutation. Sequencing of *PsTFL1a* alleles in *det* revealed point mutations that could account for the *det* phenotype.

The second gene characterized by the inventors, hereinafter designated *PsTFL1c,* segregated with the *Lf* mutations. 4 strong *Lf* alleles (*lf-a*) are deletion mutants for the *PsTFL1c* gene, whereas in 2 other *lf-a* mutants, sequencing of *PsTFL1c* showed mutations in the predicted amino acid sequence. In intermediate alleles (*Lf* or *lf*), no change was detected at the PsTFL1c protein level. The difference is due to change of *PsTFL1c* transcript level in the apex before floral initiation.

The various aspects and embodiments of the present invention are based upon the identification and functional characterisation of these genes.

Although these aspects and embodiments are illustrated hereinafter by the example of pea, they may apply to numerous other plants, in particular Angiosperms, mono- or dicotyledons. Preferred dicotyledons are Fabaceae, and more particularly Papilionoideae. The invention is of particular interest for plants belonging to any of the genus *Pisum, Glycine, Phaseolum,* or *Medicago* (alfalfa).

Therefore, the present invention concerns an isolated polypeptide hereinafter generally referred as PsTFL1 polypeptide, that is active in floral transition and/or floral development, wherein said polypeptide is selected among:
- a polypeptide hereinafter generally referred as PsTFL1a polypeptide, having at least 75%, advantageously at least 80%, preferably at least 85%, more preferably at least 90% and in a particularly preferred way at least 95% identity with the polypeptide SEQ ID NO :2;
- a polypeptide hereinafter generally referred as PsTFL1c polypeptide, having at least 75%, advantageously at least 80%, preferably at least 85%, more preferably at least 90% and in a particularly preferred way at least 95% identity with the polypeptide SEQ ID NO :4;

The inventors found that the PsTFL1a transcript is expressed only after the floral transition and is involved more specifically in floral development, while PsTFL1c transcript is expressed during both the vegetative and reproductive phase, and is involved in the control of the time of flowering. Underexpression of *PsTFL1c* in the apex before floral initiation, is responsible of early flowering, whereas overexpression may provoke late flowering.

Unless otherwise stated, sequence identity values provided herein for polynucleotides as well as for polypeptides refer to the value obtained using the BLAST suite of programs with default parameters (Altschul et al., 1997).

The invention also encompasses variants of PsTFL1 polypeptides of the invention.

This includes functional as well as functionally altered variants.

"Functional" refers to a protein having a normal biological activity. Such a protein may comprise silent mutations inducing no substantial change in its activity, and having no noticeable phenotypic effects. For instance it can comprise conservatively substitued sequences, meaning that a given amino acid residue is replaced by a residue having similar physiochemical characteristics. Examples of conservative substitutions include substitution of one aliphatic residue for another, such as Ile, Val, Leu, Ala for one another, or substitutions of one polar residue for another, such as between Lys and Arg, Glu and Asp, or Gln and Asn. Other such conservative substitutions, for example, substitutions of entire regions having similar hydrophobicity characteristics, are well known.

A "functionally altered variant" of a protein comprises one or several mutations inducing a change in its activity. Such mutations include in particular deletions, insertions, or substitutions of amino acid residues in a domain essential for the biological activity of said protein. They may result for instance in a partial or total loss of activity, or conversely in an increase of activity, or in an impairment of the response to regulatory effectors. Deletions, insertions, or non-conservative substitutions are more likely to result in a critical effect on the biological activity; however conservative substitutions may also induce a noticeable effect, if they occur at an important position of an active site of the protein.

Particular variants are those resulting from alternate mRNA splicing events, from nonsense or frameshift mutations, or from proteolytic cleavage of the PsTFL1 polypeptides. Variations attributable to proteolysis include, for example, differences in the termini upon expression in different types of host cells due to proteolytic removal of one or more terminal aminoacids from the PsTFL1 polypeptides. Variations attributable to frameshifting include, for example, differences in the termini upon expression in different types of host cells due to different amino acids of PsTFL1 polypeptides.

Non limitative examples of functionally altered variants of PsTFL1a polypeptides resulting in a partial of total loss of activity of the polypeptide include:
- a variant resulting from a point mutation at the exon-intron junction in the *PsTFL1a* gene converting the consensus splicing donor motive TGGT into a TGAT. This results in the lack of splicing of the first intron, producing a frameshift leading to a truncated protein having only the 106 C-terminal amino acids of PsTFL1a.
- a variant wherein the glutamine residue at position 127 by reference to SEQ ID NO: 2 is replaced by an arginine.
- a variant wherein the glutamic acid residue at position 104 by reference to SEQ ID NO: 2 is replaced by a lysine.

The expression of one of these functionally altered variants in a plant reduces the reproductive phase. Shortly after floral initiation the apical meristem stops growing and becomes senescent; the number of nodes bearing inflorescences on the main and lateral stems is reduced.

Non limitative examples of functionally altered variants of PsTFL1c polypeptides include:
- a variant wherein the phenylalanine, alanine, and aspartic acid residues at positions 147-149 by reference to SEQ ID NO: 4 are replaced by tyrosine;
- a variant wherein the proline residue at position 111 by reference to SEQ ID NO: 4 is replaced by a serine.

The expression of one of these functionally altered variants in a plant results in an earlier flowering time, and in a more rapid growth.

Polypeptides of the invention also include fragments of PsTFL1 polypeptides. Preferred fragments have at least 10, preferably at least 15, more preferably at least 20, and still preferably at least 50 consecutive aminoacid residues of a PsTFL1 polypeptide. Fragments that consist of sequences conserved between the polypeptides of the invention and the TFL/CEN polypeptides of the prior art are excluded.

The present invention also provides chimeric polypeptides comprising a polypeptide of the invention.

The polypeptides according to the invention can be prepared by any conventional method known in the art, for example by chemical synthesis.

They can also be prepared by culturing an host cell containing an expression vector comprising a polynucleotide encoding said polypeptide, under conditions suitable for the expression of the polypeptide, and recovering the polypeptide from the host cell culture.

They can further be purified by any of the means known in the art. Various methods of protein purification are described, e.g., in Guide to Protein Purification, ed. Deutscher, Meth. Enzymol. 185, Academic Press, San Diego, 1990; and Scopes, Protein Purification: Principles and Practice, Springer Verlag, New York, 1982.

The present invention also encompasses polyclonal and/or monoclonal antibodies capable of specifically binding to any of the polypeptides disclosed herein. Such antibodies can be produced by any conventional method. "Specific" antibodies are capable of distinguishing a given polypeptide from other polypeptides in a sample. Specific antibodies are useful, for example in purifying a polypeptide from a biological sample; in cloning alleles or homologs of a given gene sequence from an expression library; as antibody probes for protein blots and immunoassays; etc.

For the preparation and use of antibodies according to the present invention, including various antibody labelling and immunoassay techniques and applications, see, e.g., Goding, Monoclonal Antibodies: Principles and Practice, 2d ed, Academic Press, New York, 1986; and Harlow and Lane, Antibodies: A Laboratory Manual, Cold Spring Harbor Laboratory, Cold Spring Harbor, N.Y., 1988. Suitable labels for antibodies include enzymes, substrates, cofactors, inhibitors, fluorescent agents, chemiluminescent agents, magnetic particles and the like.

The present invention also concerns isolated polynucleotides selected from a group consisting of :
a) polynucleotides encoding a PsTFL1 polypeptide of the invention, and their complements;
b) polynucleotides which hybridizes under high stringency conditions with a polynucleotide a), and their complements;
c) fragments of at least 10, preferably at least 15, more preferably at least 20, still more preferably at least 50, and advantageously at least 100 consecutive nucleotides of a polynucleotide mentioned in a) or b) above.

A "polynucleotide encoding a PsTFL1 polypeptide" is any polynucleotide comprising the information allowing the translation into said polypeptide. It may consist of an isolated coding sequence (CDS); it may also consist of a whole length mRNA comprising said CDS, or of the corresponding cDNA or genomic DNA.

In particular the invention encompasses isolated polynucleotides comprising a *PsTFL1a* or *PsTFL1c* gene, including the coding sequence, the 3' and 5' UTR, the introns, and the promoter region.

Preferred fragments of polynucleotides a) are specific fragments thereof. "Specific fragments" refers to polynucleotides having a sequence that is found in a polynucleotide encoding a PsTFL1 polypeptide of the invention, and is not found in polynucleotides encoding related polypeptides of the prior art. Preferred fragments of polynucleotides b) are fragments specifically hybridizing with polynucleotide a) or specific fragments thereof.

"Specifically hybridizing" refers to polynucleotides that can hybridize, under high stringency conditions, with polynucleotides encoding a polypeptide of the invention, without hybridizing with polynucleotides encoding related polypeptides of the prior art.

According to a preferred embodiment, a polynucleotide of the invention is selected among:
- a polynucleotide comprising a nucleotide sequence having at least 75%, advantageously at least 80%, preferably at least 85%, more preferably at least 90%, and in a particularly preferred way at least 95% identity with SEQ ID NO : 1;
- a polynucleotide comprising a nucleotide sequence having at least 75%, advantageously at least 80%, preferably at least 85%, more preferably at least 90%, and in a particularly preferred way at least 95% identity with SEQ ID NO : 3,
   as well as their fragments, as defined above, and polynucleotides which hybridizes under high stringency conditions with said polynucleotides or fragments.

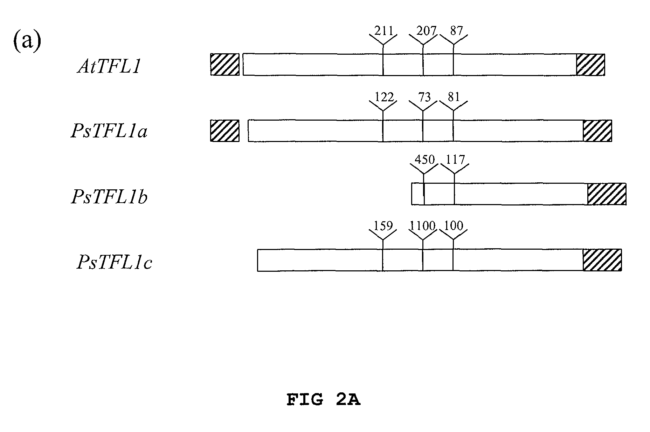

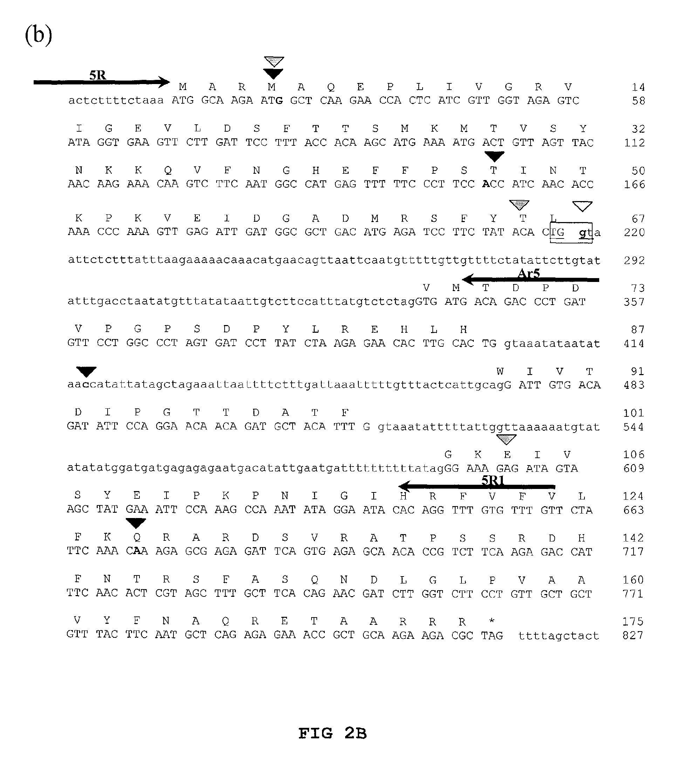

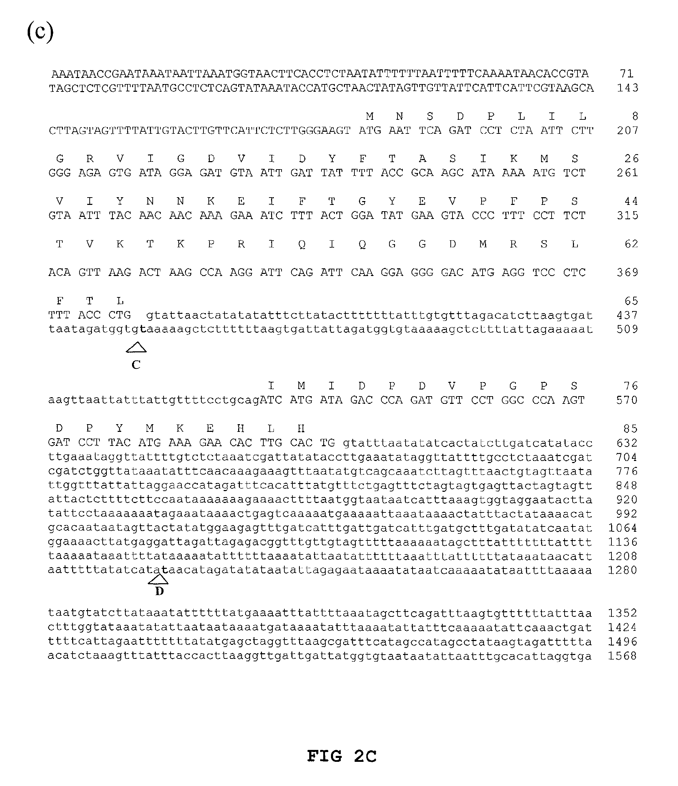

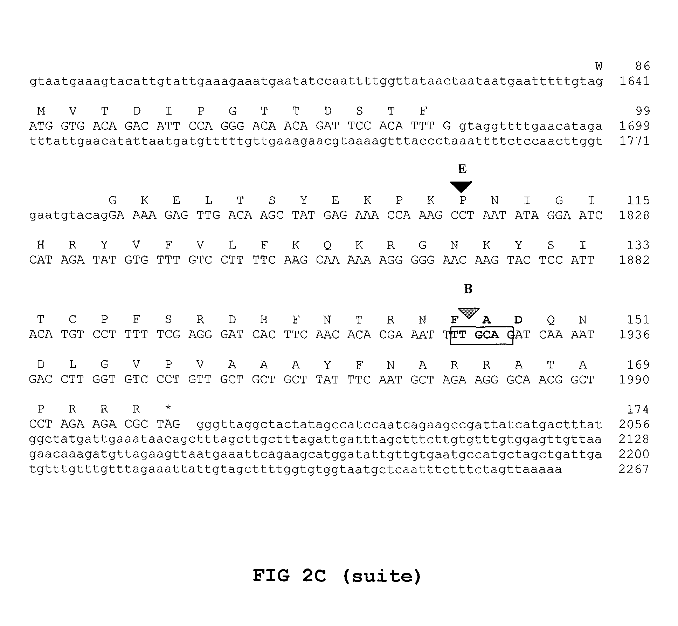

Examples of polynucleotides are for instance the genomic DNA sequences of *PsTFL1a* and *PsTFL1c* represented in Figure 2 (b and c), and in the enclosed sequence listing as SEQ ID NO:5 and SEQ ID NO:6.

The present invention also encompasses polynucleotides that can be obtained from a *Pisum sativum* cDNA or genomic DNA library by screening said library under high stringency conditions with primers or probes having a nucleotide sequence selected from a group consisting of SEQ ID NO : 1, SEQ ID NO : 3, fragments thereof, or their complementaries. It also encompasses polynucleotides that can be obtained by screening with said primers or probes cDNA or genomic DNA libraries of Papilionoideae, in particular those belonging to any of the genus *Pisum, Glycine, Phaseolum,* or *Medicago.* In these cases, lower stringency conditions will be used.

The terms "high stringency conditions" refers to conditions under which a probe will hybridize to its target sequence to a detectably greater degree than to other sequences (e.g., at least 2-fold, preferably at least 5-fold over background).

Nucleic-acid hybridization is affected by such conditions as salt concentration, temperature, or organic solvents, in addition to the base composition, length of the complementary strands, and the number of mismatched bases between the hybridizing nucleic acids. Generally, stringent conditions are selected to be about 5°C lower than the melting point (Tₘ) for the specific sequence and its complement at a defined ionic strength and pH.

As an illustration, the high stringency conditions that may be used in order to specifically detect a polynucleotide according to the present invention are advantageously the following:
Prehybridization and hybridization are performed at 68°C in a mixture containing:
   5X SSPE (1X SPE is 0.18 M NaCl, 10mM NaH₂PO₄.);
   5X Denhardt's solution;
   0.5% (w/v) sodium dodecyl sulfate (SDS); and
   100 µg ml⁻¹ salmon sperm DNA.

The washings are performed as follows:
Two washing at laboratory temperature for 10 min in the presence of 2X SSPE and 0.1% SDS;
One washing at 68°C for 15 min. in the presence of 1X SSPE and 0.1% SDS; and
One washing at 68°C for 15 min. in the presence of 0.1X SSPE and 0.1% SDS.

In contrast, low stringency and moderate stringency conditions differ from stringent conditions in that steps b) and c) are respectively performed at 50°C, or 55°C-60°C.

Polynucleotides of the invention as well as fragments thereof may be used to prepare probes or primers for detecting and/or amplifying a nucleic acid sequence encoding a polypeptide of the invention.

They may eventually be labeled, for instance with a radioactive element (³²P, ³⁵S, ³H, ¹²⁵I) or by a non-isotopic molecule (for example, biotin, acetylaminofluorene, digoxigenin, 5-bromo-desoxyuridin, fluorescin).

Methods for preparing and using probes and primers are described, for example, in Sambrook et al., Molecular Cloning: A Laboratory Manual, 2nd ed., vol. 1-3, ed. Sambrook et al., Cold Spring Harbor Laboratory Press, Cold Spring Harbor, NY, 1989; Current Protocols in Molecular Biology, ed. Ausubel et al., Greene Publishing and Wiley-Interscience, New York, 1987 (with periodic updates); and Innis et al., PCR Protocols: A Guide to Methods and Applications, Academic Press: San Diego, 1990.

Said probes and primers are also part of the invention. In particular, the invention encompasses any set of primers comprising at least one primer consisting of a fragment of a polynucleotide of the invention.

The invention also comprises recombinant DNA constructs comprising a polynucleotide of the invention. This includes in particular:
- expression cassettes comprising a polynucleotide of the invention (in sense or antisense orientation) under transcriptional control of a suitable promoter;
- recombinant vectors comprising a polynucleotide or an expression cassette of the invention.

These DNA constructs can be obtained and introduced in a host cell or organism by the well-known techniques of recombinant DNA and genetic engineering.

A large choice of promoters suitable for expression of heterologous genes in varied hosts is available in the art.

In particular, promoters capable to direct transcription in plant cells include a large variety of promoters that can be obtained for instance from plants, plant viruses, or bacteria such as *Agrobacterium.* They include constitutive promoters, i.e. promoters which are active in most tissues and cells and under most environmental conditions, tissue or cell specific promoters which are active only or mainly in certain tissues or certain cell types, and inducible promoters that are activated by physical or chemical stimuli.

Non-limitative examples of promoters that are commonly used in plant cells are the cauliflower mosaic virus (CaMV) 35S promoter, the Nos promoter, the rubisco promoter.

An example of tissue specific promoters active in apical meristem is the *TFL1* promoter; examples of tissue specific promoters active in floral meristems are *Agamous,* or *LEAFY* promoters.

One may also use the endogenous promoter of any of the *PsTFL1a* or *PsTFL1c* genes, eventually altered in order to up-regulate or down-regulate gene expression.

The invention also provides a prokaryotic or eukaryotic host cell genetically modified by a polynucleotide of the invention. Preferably, the host cell is a plant cell.

The polynucleotide may be transiently expressed; it can also be incorporated in a stable extrachromosomal replicon, or integrated in the chromosome.

Introduction of a polynucleotide of the invention into host cells can be effected by means well known in the art Generally, said polynucleotide is incorporated in a DNA construct of the invention that is introduced in the cell by any appropriate method, such as transfection, microinjection, electroporation, transduction, ballistic introduction, and the like. In the case of plant cells, one can also use *Agrobacterium* mediated transformation.

The invention also include plant tissues or organs, or tissue cultures comprising transformed plant cells of the invention.

The transformed cells, tissues, or organs of the invention can be used to regenerate transgenic plants. Many methods of cell transformation and plant regeneration for numerous plant species are known in the art. For instance, a method for genetically transforming pea plants is disclosed in the US Patent 5,286,635.

The invention also encompasses transgenic plants comprising a polynucleotide of the invention. "Transgenic" is used herein to include any plant having a genotype modified by the presence of a polynucleotide of the invention.

This includes in particular:
- transgenic plants genetically modified by a polynucleotide of the invention expressing a functional PsTFL1 polypeptide;
- transgenic plants genetically modified by a polynucleotide of the invention expressing a PsTFL1 variant, having an increased or conversely a decreased activity;
- transgenic plants genetically modified by a polynucleotide of the invention inducing the silencing of a *PsTFL1* gene.

Preferably, the polynucleotide is stably integrated within the genome of the plant and is passed on to successive generations. Descendants of the initial transgenic plants are thus also part of the invention, provided that they remain genetically modified by a polynucleotide of the invention.

The invention also comprise parts from the plants of the invention such as flowers, seeds, leaves, branches, fruit, provided that these parts comprise cells genetically modified by a polynucleotide of the invention.

The invention also provides a method for regulating the floral transition and/or floral development in a plant wherein said method comprises modulating the expression of at least one PsTFL1 polypeptide of the invention in said plant.

For instance:
- the expression of PsTFL1a polypeptide in a plant can be increased for the purpose of lengthening the reproductive phase;
- the expression of PsTFL1c polypeptide in a plant can be increased for the purpose of delaying the flowering;
- the expression of both PsTFL1a and PsTFL1c polypeptide in a plant can be increased for the purpose of extending the vegetative and reproductive phases.
- the expression of PsTFL1a polypeptide in a plant can be decreased for the purpose of reducing the number of flowers, avoiding a competition between flowers and seed development;
- the expression of PsTFL1c polypeptide in a plant can be decreased for the purpose of obtaining an earlier flowering time and a more rapid growth;
- the expression of both PsTFL1a and PsTFL1c polypeptides in a plant can be decreased for the purpose of obtaining an earlier flowering time, and plants bearing only few flowers.

The expression of a PsTFL1 polypeptide in a plant can be increased, for instance, by expressing in said plant a DNA construct of the invention producing said polypeptide, or by altering the corresponding *PsTFL1* endogenous promoter in order to up-regulate gene expression.

Conversely, a decrease of the expression of a PsTFL1 polypeptide can be obtained for instance by expressing in said plant a polynucleotide of the invention inducing the silencing of a *PsTFL1* gene, for instance by antisense inhibition or cosuppression, as described by way of example in US Patents S.N. 5,190,065 and 5,283,323. It is also possible to use ribozymes targeting PsTFL1 mRNA, or to alter the *PsTFL1* promoter in order to down-regulate gene expression.

According to a particular embodiment of the invention, the modulation of the level of PsTFL1a and/or PsTFL1c may be effected in a plant having at least one other mutation. For instance, decreasing the level of PsTFL1a polypeptide in a plant having a mutation affecting basal branching such as *rms* mutants [for instance *rms* 6 mutant has an increased basal branching (Rameau et al., Physiol Plant, 115(3): 458-467, 2002)], may give plants bearing several branches and only few nodes bearing pods, flowering almost at the same time with less competition between growing pods.

The invention also provides means for evaluating the flowering characteristics of a plant.

According to a first aspect the invention provides:
- a method for identifying an allele of *PsTFL1a* or an allele of *PsTFL1c* associated with a given flowering phenotype, wherein said method comprises isolating a nucleic acid fragment comprising the *PsTFL1a* or *PsTFL1c* gene or a portion thereof from at least one plant expressing said phenotype and sequencing said fragment;

The invention further provides:
- a method for identifying polymorphisms associated with flowering phenotype, in the *PsTFL1a* gene, wherein said method comprises identifying, as described above, at least two different alleles of *PsTFL1a* associated with different flowering phenotypes and comparing the sequences of said alleles;
- a method for identifying polymorphisms associated with flowering phenotype, in the *PsTFL1c* gene wherein said method comprises identifying, as described above, at least two different alleles of *PsTFL1c* associated with different flowering phenotypes and comparing the sequences of said alleles.

Once a polymorphism has been identified, reagents and kits allowing the routine detection of said polymorphism can be designed. Commonly used reagents are nucleic acid probes, or restriction enzymes, or PCR primers, or combinations thereof. The choice of a reagent or of a combination of reagents depends of the nature of the polymorphism.

Preferred kits and reagents are those comprising a set of primers allowing specific PCR amplification of a DNA segment spanning the polymorphic locus. For microsatellites and insertion/deletion polymorphisms, PCR primers may be sufficient, since the allelic forms of the polymorphism may be differentiated by the size of the amplification product. In the case of single nucleotide polymorphisms (SNP), one will generally also use a restriction enzyme, allowing to differentiate the allelic forms by the presence or size of restriction fragments.

The invention also provide a method for testing a plant for its flowering properties wherein said method comprises detecting whether an allele of the *PsTFL1a* gene associated with a given flowering phenotype, or an allele of the *PsTFL1c* gene associated with a given flowering phenotype, is present in said plant.

According to a preferred embodiment of the method of the invention, said flowering phenotype is short reproductive phase, and production of only few flowers and the allele of the *PsTFL1a* gene associated with said phenotype expresses no PsTFL1a polypeptide, or a lower level of PsTFL1a polypeptide, or a less active PsTFL1a polypeptide than the wild type allele, or an inactive PsTFL1a polypeptide.

Examples of alleles resulting in said phenotype are:
- alleles having a deletion of all or part of the *PsTFL1a* gene;
- alleles having a mutation resulting in the expression of a less active or inactive variant of PsTFL1a;
- alleles having a mutation in the *PsTFL1a* promoter, resulting in a lower expression of PsTFL1a polypeptide:

- alleles that are splicing variants of the *PsTFL1a* gene.

For instance, some alleles found by the inventors to be associated with said phenotype are:
- an allele having a mutation at the first exon-intron junction, resulting in the conversion of the consensus splicing donor motive TGGT into TGAT;
- an allele having a mutation resulting in the replacement of the glutamine residue at position 127 of PsTFL1a polypeptide by an arginine;
- an allele having a mutation resulting in the replacement of the glutamic acid residue at position 104 of PsTFL1a polypeptide by a lysine.

Alternatively, said flowering phenotype is a long reproductive phase, and the allele of the *PsTFL1a* gene associated with said phenotype expresses a higher level of PsTFL1a polypeptide, or a more active PsTFL1a polypeptide than the wild type allele.

According to another preferred embodiment of the method of the invention, said flowering phenotype is early flowering and the allele of the *PsTFL1c* gene associated with said phenotype expresses no PsTFL1c polypeptide, or a lower level of PsTFL1c polypeptide, or a less active PsTFL1c polypeptide than the *Lf* allele or an inactive PsTFL1c polypeptide.

Examples of alleles resulting in said phenotype are:
- alleles having a deletion of all or part of the *PsTFL1c* gene;
- alleles having a mutation resulting in the expression of a less active or inactive variant of PsTFL1c;
- alleles having a mutation in the *PsTFL1c* promoter, resulting in a lower expression of PsTFL1c polypeptide;
- alleles that are splicing variants of the *PsTFL1c* gene.

Some alleles found by the inventors to be associated with said phenotype are, by way of example:
- alleles having a deletion of the *PsTFL1c* gene resulting in the lack of expression of PsTFL1c polypeptide;
- an allele having a 6 pb deletion in the in the coding region of the *PsTFL1c* gene resulting in the replacement of the phenylalanine, alanine, and aspartic acid residues at positions 147-149 of PsTFL1c polypeptide by a tyrosine;
- an allele having a C→T substitution at position 1815 of the *PsTFL1c* gene (by reference to SEQ ID NO: 3) resulting in the replacement of the proline residue at position 111 of PsTFL1c polypeptide by a serine.
- alleles having a level of *PsTFL1c* transcript lower (between 5 to 20 times less) than wild type lines.
   Alternatively, said flowering phenotype is late flowering, and the allele of the *PsTFL1c* gene associated with said phenotype expresses a higher level of PsTFL1c polypeptide, or a more active PsTFL1c polypeptide than the *Lf* allele.
   Foregoing and other objects and advantages of the invention will become more apparent from the following detailed description and accompanying drawings.
   It is to be understood that both the foregoing general description and the following detailed description are exemplary and explanatory only and are not restrictive of the invention, as claimed.

### DESCRIPTION OF THE DRAWINGS.

### Figure 1:

Schematic representation of different architecture of plants and their *TFL1* homologue mutants. Black arrow represents shoot apical meristem, white circle represents flowers, and black circle represents stub (apical meristem with epidermal hairs).

### Figure 2:

Sequence analysis of pea *TFL1* homologues
(a) Schematic diagram of the exon(boxes)/intron organization in *TFL1* from Arabidopsis (Bradley, 1997), *PsTFL1a, b* and *c*. Numbers give the intron size in base pairs (bp).
(b) and (c) Organization of the *PsTFL1a* and *PsTFL1c,* respectively, genomic sequence from the cultivar Térèse. Intronic sequences are in lower case and exonic in upper case. (b) Changes observed in the *det* lines are represented by triangles (*det-1* in white, *det-2* in black and *det-3* in gray). Black arrows represent the primers used to detect splicing in *det-1* line. (c) Changes observed in the *lf-a* lines are represented by triangles (HL76 in black and Wt11795 in grey). Silent substitutions present in some other *Lf* lines are represented by white triangles. The letters B, C, D and E refer to the haplotypes defined in Table I. The box corresponds to the 6 bp deletion in Wt11795.

### Figure 3:

(a) Alignment of the predicted amino acid sequences of *TFL1, PsTFL1a, b* and *c*, *SP, CEN, FT.*
(b) Phylogenic tree from the predicted amino acid sequences of the *TFL1* family genes in different plant species: *Arabidopsis* and of the rat PEBs.

### Figure 4 :

Genomic Southern of wild type peas (Térèse (Te) and Paloma (Pa)) and *det1* mutant (JI2121). Size of the fragment is represented in kilobase (kb) on the left.

### Figure 5 :

Comparison of the localization of *PsTFL1a* and *det* on pea genetic map. (a) and (c) mapping of *det* in a F2 population obtained by the cross of Térèse (Det/Det) and JI2121 (*det1*/*det1*).
(b) mapping of *PsTFL1a* in the recombinant inbreed line between Térèse and 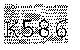 (Laucou, 1998) between the RAPD markers K3-3000 and D3-1100. Common markers between the 3 maps are linked with dot lines.

### Figure 6:

(a) Alignment of the predicted amino acid sequence of *PsTFL1a* genes in wild type pea (cultivar Térèse and Paloma) and *det* lines (*det-1*(JI2121), *det-2*(JI1358), *det-3*(JI3100))
(b) Results of PCR performed on root cDNA from Paloma (Pa), *det1* (JI2121), water (H₂O) and genomic DNA (gDNA) as control. Arrows indicate the size expected for the cDNA if intron 1 is spliced (cDNA) or not spliced (cDNA + intron 1).
(c) Alignement of the predicted amino acid sequence of *PsTFL1c* genes in wild type (Térèse) and *lf*-a lines (HL76 and Wt11795). Letters with a black background represent different residues, and letters with gray background similar residues.

### Figure 7:

Analysis of the *PsTFL1c* gene by genomic Southern blot in different *Lf* lines.

### Figure 8:

Analysis of the *PsTFL1c* transcript level in different *Lf* lines by Real Time PCR.

### Figure 9:

Analysis of *PsTFL1* gene expression in different tissues by RT-PCR: root (R), dormant node (N), internode (IN), leaf (L), apex from vegetative plants (A), flower bud (FB), flowers (F), apex during the floral transition under short day (SD) or long day (LD) conditions (A1 represents apex before the floral transition, A2 apex after floral transition and before flowers open and A3 apex after flowers open).

Controls : genomic DNA (gDNA); water (0).

### EXAMPLES

### Plant material and growth conditions

*det* lines were obtained from the John Innes Pisum Germaplast collection. *det1* line corresponds to JI2121, and was obtained by mutation of the cultivar Paloma (Swiecicki, 1987). *det2* line (JI1358) is a spontaneous mutant and *det3* line (JI3100) was obtained by mutation of the line SG (Berdnikov, 1999). SG line is not yet available and a close relative line was chosen (SGE, JI3023). Other used wild type peas are Térèse and Torsdag cultivar.

Plants were grown in the greenhouse conditions in aeroponic bac. For the floral initiation experiment, the plants were grown in cabinets at 20°C during the day and 15°C during the night, illuminating by mercury vapor lamps (135 mE/m2/sec) in a (1:1:1) mixture of sphagnum: clay, under short day (12 h light) or long day (18 h light) conditions. Grafts (2 plants per pot) were made epicotyl to epicotyl as described by Murfet (1971).

### EXAMPLE 1: CLONING AND ISOLATION OF TFL1 HOMOLOGUES IN PEA.

The *TFL1* homologues were isolated from genomic DNA in Térèse by using the following primers: 
TFL1-1 (SEQ ID NO:7 ):
5' ATG GGG AGA GTG AT(ACT) GG(AG) GA(AT) G 3';
 
TFL1-2 (SEQ ID NO:8 ):
5' TCA CTA GG(AG) CC(AT) GGA ACA TC(AT) GG 3';
 
TFL1-3 (SEQ ID NO:9 ):
5' GAT GTT CC(AT) GG(AT) CCT AGT GA(CT) CC 3'
 
TFL1-5 (SEQ ID NO:10 ):
5' CTT GCA GC(AG) GT(CT) TC(CT) CT(CT) TG 3'
 The PCR conditions were 35 cycles at 94°C, 60s; 55°C, 60s and 72°C 2min.

One band of 450 bp (band1), using the primer combination TFL1-1 and TFL1-2, and two bands (band 2 and 3 of respectively 450 and 850 bp length) using the combination TFL1-3 and TFL1-5, were isolated and sequenced.

The 3 sequences were similar to *TFL1* and *CEN.* To obtain the full-length sequence, 3' and 5' RACE PCR were performed on cDNA from seedlings and flowers using the kit and the recommendations of the supplier (Life Technology, Rockville, MD)

Complete sequences were obtained for the band 1 and 2, which were called respectively *PsTFL1c* and *a*. Only the 3' part of the gene corresponding to band 3 was isolated and denominated *PsTFL1b. PsTFL1b* therefore represents a partial sequence.

*PsTFL1a* is predicted to encode a protein of around 174 amino acid with 3 introns according to the computer programme Eugene (Figure 2) Prediction by Genscan selects another methionine (methionine at position 3) as start codon. *PsTFL1c* is predicted to encode a protein of 173 amino acids, and as for *PsTFL1a* the position of the introns is conserved between *Arabidopsis* and pea (Figure 2a). PsTFL1a and c present 70 % identity at the protein level. For the partial *PsTFL1b* clone, 2 introns are present and their positions are also conserved (Figure 2a).

The 3 sequences are related to the *TFL1* gene family.

TFL1 is 72%, 73% and 65 % identical at the protein level with PsTFL1a, PsTFL1b and PsTFL1c, respectively.

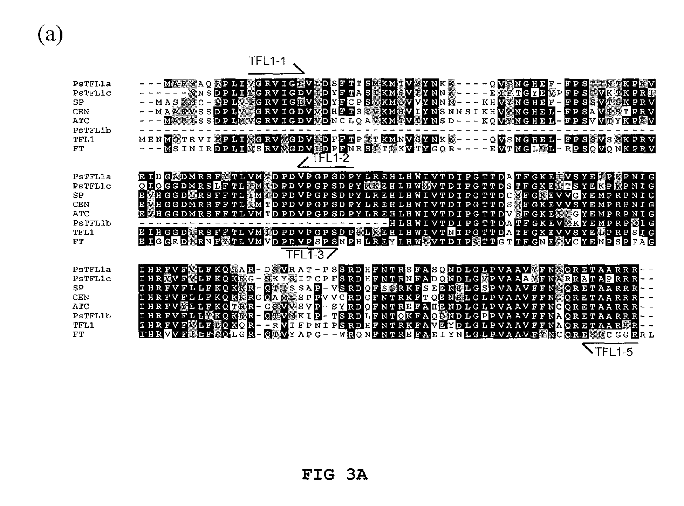

Alignment of the predicted amino acid sequences of *TFL1* (Bradley, 1997), *PsTFL1a, b* and *c*, *SP* (Pnueli, 1998), *CEN* (Bradley, 1996), *FT* (Kardailsky, 1999; Kobayashi, 1999) was performed with multialign software (Corpet, 1988). Large conserved domains are found between CEN, TFL1, SP, PsTFL1a, PsTFL1b and PsTFL1c as shown on the sequence alignment (Figure 3a). The structure of the genes is conserved between the species according to predicted exon/intron bundaries (Figure 2a).

A phylogenic tree from the predicted amino acid sequences of the *TFL1* family genes in different plant species: Arabidopsis (*TFL1* (Bradley, 1997), *FT* (Kardailsky, 1999; Kobayashi, 1999), *TSF* (accession number AB027506), *BFT* (accession number AB016880), *ATC* (Mimida, 2001) *and MTF* (accession number AF147721)), tomato (*SP,* Pnueli, 1998), tobacco (*CET2, CET4 and CET1*, Amaya, 1999), snapdragon (*CEN,* Bradley, 1996), rice (*FDR1 and FDR2* (accession number AAD42895 and AAD42896 respectively)), *Medicago truncatula* (*MtTFL1a and MtTFL1b* (from MtGI EST TC32803 and TC34801 respectively)), garden pea (*PsTFL1a, b and c*) and *Lolium perenne* (*LpTFL1,* Jensen, 2001) and from the rat PEBs (Accession number X75253) was made using Multialign software.

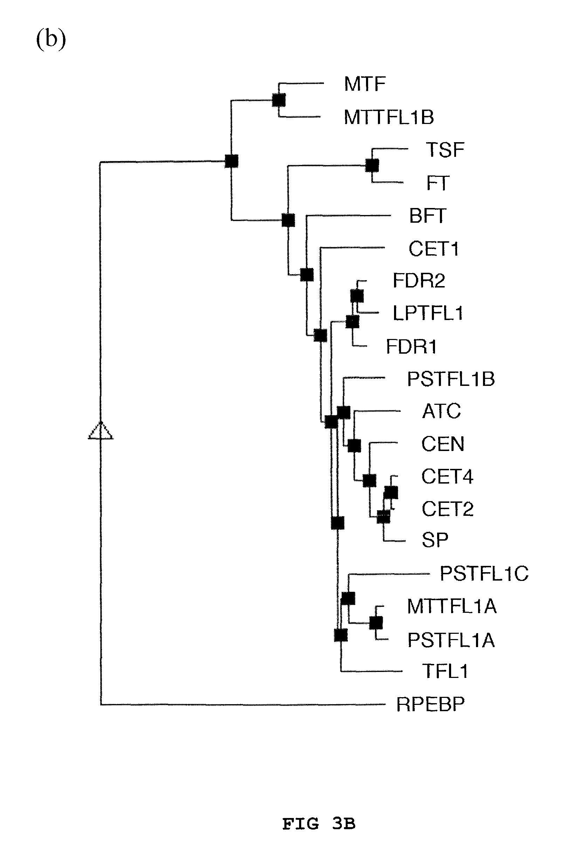

The results are shown on Figure 3b. Based on amino acid similarity, different groups of genes can be proposed. As shown previously *TFL1* and *CEN* are not closely related (Mimida, 2001). *TFL1* is closely related to *PsTFL1a* and c and a *Medicago truncatula* EST. *PsTFL1b* belongs to the group of genes represented by *CEN, SP* or *ATC. PsTFL1b* is a partial sequence, and results should be take carefully.

A third group of genes is represented by *TFL1* sequences from grass (rice and *Lolium perenne*). Genes (such as FT) or other members of the *Arabidopsis TFL1* gene family) are more distant and cannot be grouped with the *TFL1* sequences.

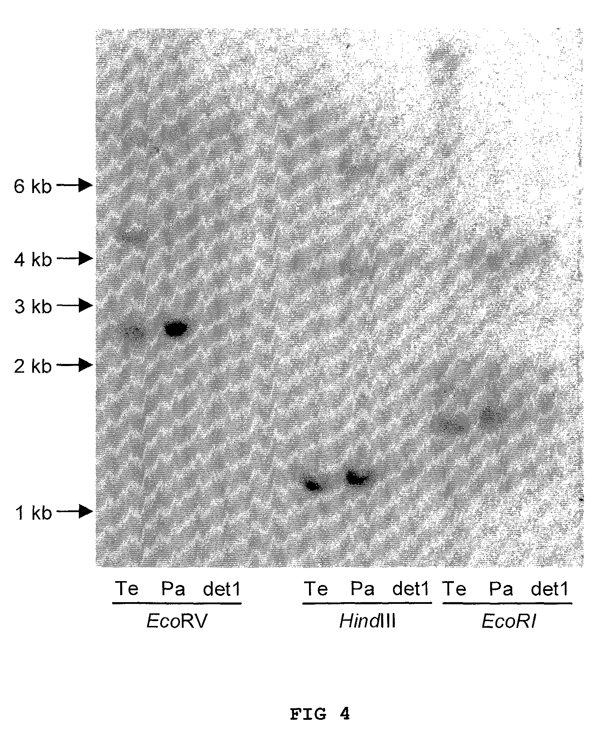

To evaluate the number of *TFL1* homologues in pea, a Southern blot, probed with *PsTFL1a,* was performed. The genomic DNA was digested with *Eco*RV, *Hind*III and *Eco*RI restriction enzymes. The blot was hybridized with a probe corresponding to the *PsTFL1a* gene. The results are shown on Figure 4.

Only 3 bands are obtained with *Hind*III and *Eco*RV digestion and 4 bands with *Eco*RI. In the case of *Eco*RI, an *Eco*RI site is present in *PsTFL1b* sequence, which can explain the presence of a fourth band. We concluded that only 3 *TFL1* homologues are present, but we can not exclude the possibility that more distantly related genes are present in pea, that can not be detected by hybridization due to low homology at the DNA level.

### EXAMPLE 2: PsTFL1a COSEGREGATES WITH THE DET LOCUS AND PSTFL1C WITH LF ON THE PEA GENETIC MAP.

Mapping was done on two different mapping populations obtained respectively by the cross of the pea lines Térèse X K586 (Laucou, 1998) for *PsTFL1a* and JI281 X JI399 (Ellis, 1992) for *PsTFL1b* and *c*. Polymorphism was searched between the parents of mapping population to develop derived PCR markers such as dCAPS (Cleave Amplified Polymorphism Sequence, Michaels, 1998 ; Neff, 1998).

One single nucleotide polymorphism (SNP) between Térèse and K586 was detected for *PsTFL1a,* and a dCAPS was developed by using the following primers: 
TFL2 (SEQ ID NO:11):
5' GAA CAC TTG CAC TGG TAA ATA TAA TAG A 3';
 
TFLR (SEQ ID NO:12):
5' TGT AGC ATC TGT TGT TCC TGG 3';
 and digestion of the PCR product by the *Hinf*I enzyme.

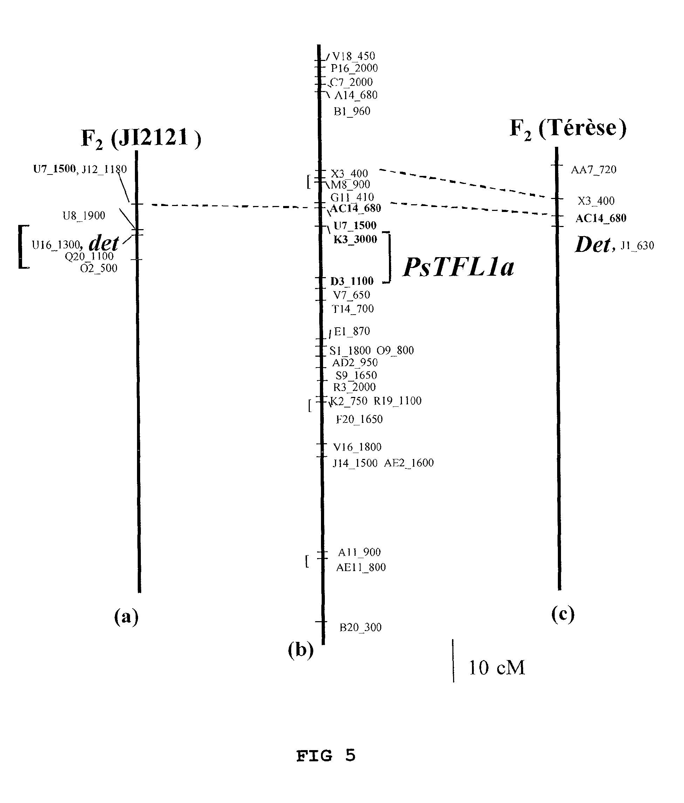

The *PsTFL1a* gene was mapped to linkage group V between two RAPD markers, K3-3000 and D3-J1000 (Figure 5). Previous mapping of the *det* mutation localized the gene on the same linkage group at the vicinity of the same RAPD markers (cross between the cultivar Térèse (Det/Det) and JI2121 (det/det), Figure 5). Analysis of a F2 population derived from Térèse and JI2121 revealed no recombination between Det and PsTFL1a in 120 individuals (Data not shown). *PsTFL1a* was therefore a candidate for the gene resposible for the *det* mutation.

In the same way, polymorphisms were found between JI281 and JI399 for *PsTFL1b* and *c*.
CAPS markers were developed
- for *PsTFL1b:*
   primer pairs : 
TFLb32(SEQ ID NO:13):
5' GAA ATA TGA AAT GCC ACG TCC 3')
 
TFL1bR1 (SEQ ID NO:14):
5' ACA AAC TAG AAC AAC AAC AAC CC 3');
 restriction enzyme: *Hha*I)
- for *PsTFL1c :*
   (primer pairs : TFL1-1 and TFL1-2;
   restriction enzyme *HpyF44*III).

*PsTFL1b* maps to linkage group III in a region where no mutant is described. *PsTFL1c* maps to linkage group II. It segregates with the B5/9 markers (Ellis, 1992) close to the *LF* locus. Therefore, *PsTFL1c* is a good candidate for the *Late flowering* gene.

### EXAMPLE 3: PSTFL1A IS THE ORTHOLOGUE OF TFL1 AND CORRESPONDS TO THE DET MUTATION IN PEA.

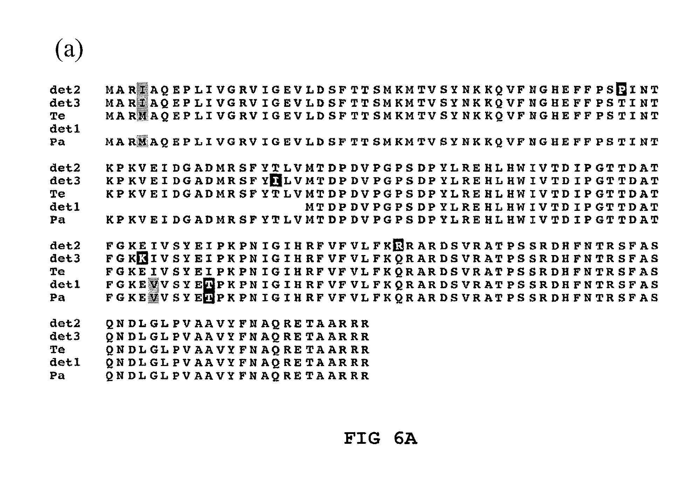

To confirm the mapping data result, the *PsTFL1a* gene from 3 different *det* mutants (*det-1*(JI2121), *det*-*2*(JI1358), *det*-*3* (JI3100)) and from the corresponding wild type lines (cultivar Térèse and Paloma) was sequenced. Alignment of the predicted amino acid sequences, performed using Clustal W software, (Thompson, 1994) is shown in Figure 6a.

In the *det-1* mutant (JI 2121), a point mutation was found at the first exon-intron junction, converting the consensus splicing donor motif TGGT into a TGAT (Figure 2b).

To confirm that the first intron is not spliced in *det-1,* RT-PCR was performed on root RNA from *det-1* and Paloma, using two pairs of primers surrounding the intron 1 (5R*AR5 and 5R*5R1, see Figure 2 for the position of the primers on the sequence).

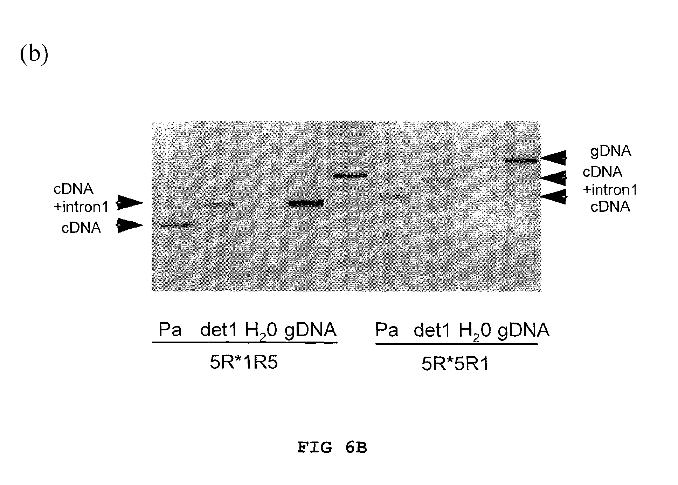

The results are shown in Figure 6b. The cDNA amplified from *det-1* is longer than those amplified from Paloma. The size corresponds to the intron 1 size (around 210bp). The second intron is well spliced as shown by amplification with the primers surrounding the intron1 and 2. The non-splicing of the first intron results in a truncated protein. This large deletion suggests that the det phenotype results from a mutation in *PsTFL1a.*

The *det-2* plant (JI 1358) corresponds to a spontaneous mutant (from the John Innes Stock Center). When the *PsTFL1a* sequence from *det-2* was compared to Térèse sequence, several point mutations were found, which may correspond to natural polymorphism in *det-2*. Some SNP (single nucleotide polymorphism) are present in the introns or UTR (untranslated transcript regions). At the protein level, 3 changes were detected in the amino-acid sequences. Methionine at position 4, threonine at position 47 and glutamine at position 127 are changed respectively into isoleucine, proline and arginine (Figure 6a). The methionine and threonine are not conserved when plant TFL1 sequences are aligned (see Figure 3a), whereas the glutamine at position 127 is a well-conserved amino acid found in all *TFL1* sequences, even in distant sequences such as *FT* or animal PBP sequences. The glutamine may therefore represent an important amino acid, and a mutation at this point in the coding sequence can account for the *det-2* phenotype.

The *det-3* mutant was obtained by mutation of the SG line (Berdnikov, 1999). Since the SG line is not available, a close relative line (SGE, JI3023) was chosen for comparison. Numerous differences between SGE and *det-3* at the *PsTFL1a* locus (SNP and microsatellite polymorphism, data not shown) were found. Three differences in the protein sequence were detected (Figure 6a).

One is in common with *det-2*: a methionine at position 4 is changed into a similar amino acid, isoleucine.

The second is at position 66 where a non-conserved threonine is changed into isoleucine. The same change is found in the *Arabidopsis tfl1-14* mutant. This mutation in *Arabidopsis* is responsible of the *tfl1* phenotype (Ohshima, 1997). The third change is at position 104 where glutamic acid is converted into lysine. Glutamic acid is found in all the *TFL1* related sequences in plants, and therefore may represent an important amino acid. A mutation at that position is likely to result in a non-functional PsTFL1a protein, and account for the *det-3* phenotype.

### EXAMPLE 4: PSTFL1C CORRESPONDS TO THE LATE FLOWERING GENE

In order to verify the hypothesis that *PsTFL1c* may correspond to the *LF* gene, we studied the *PsTFL1c* gene in different lines. *LF* presents 4 natural alleles: *Lf-d, Lf, lf* and *lf-a* (Taylor, 1993). *lf-a* lines present the stronger phenotype with the earliest flowering node (node 5); *Lf-d* lines have the latest flowering, *Lf,* and *lf* have intermediate phenotypes.

The *PsTFL1c* gene from different lines (listed in Table I below) was amplified by PCR using the following primer combination:

TFL1cR13 (SEQ ID NO:15):
5' CTC GTT TTA ATG CCT CTC AGT A 3';

TFL1cR6 (SEQ ID NO:16):
5' GAG CAT TAC CAC ACC AAA AGC 3';

Then, the PCR products were sequenced and 5 haplotypes (according to the sequence) were defined: A is the Térèse reference sequence, B present a deletion of 6 bp, C and D are silent substitutions in the intronic region and E a non silent substitution at position 1815. Positions of the substitutions and deletions are represented in Figure 2c.

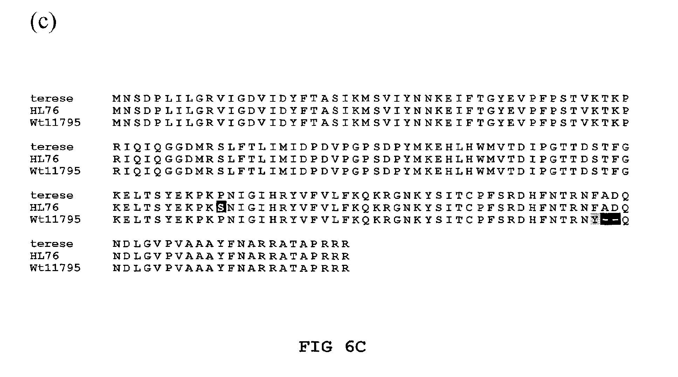

Figure 6c shows the alignement (performed using Clustal W software) of the predicted amino acid sequence of *PsTFL1c* genes in the wild type line Térèse and the 2 *lf-a* lines HL76 and Wt11795.

In HL76, a 6bp deletion was found (compared to the HL75 progenitor line) in the coding region leading to a change in the amino acid sequence. 3 amino acids at position 147 (phenylalanine (F), alanine (A) and aspartic acid (D)) are replaced by tyrosine. It is to be noticed that the phenylalanine is conserved in SP, CEN and TFL1 amino acid sequences (Figure 3a).

In the Wt11795 *lf-a* line, a substitution (C changed into T) at position 1815 is responsible for the replacement of the proline at position 111 by a serine. The proline at this position is conserved in all TFL1-related plant sequences (Figure 3a). These two changes in the amino acid sequence of HL76 and Wt11795 may give rise to a non-active PsTFL1c protein, that can account for the *lf-a* phenotype.

Lines having intermediate phenotype (*lf* or *Lf*) were also sequenced. The PsTFL1c amino-acid sequences are the same for all these lines. Only silent substitutions in the introns were detected (see Figure 2c). These substitutions may represent natural polymorphisms present in the pea population. It means that no change in the PsTFL1c amino acid sequence can be associated with the *Lf* intermediate phenotype. Plants having *Lf-d, Lf* or *lf* alleles, produce the same PsTFL1c protein.

In 4 *lf-a* lines (HL7, XVIII/17, Wt11796 and K2), the *PsTFL1c* gene cannot be amplified by PCR.

Genomic Southern blot of different *LF* lines was performed. DNA was extracted from leaves according to the protocol described in Laucou, (1998). 10 µg of genomic DNA was digested by *EcoR*I and loaded on 0.7% agarose gel. Blotting was performed according to the recommendation of the membrane supplier (Biotrans Nylon Membrane, ICN, Ref 810-2000). The blot was hybridized with a *PsTFL1c* probe in Church and Guilbert buffer at 65°C overnight (Church, 1984). Washing was done at 65°C according to Sambrook, 1989 (2 SSC, 0,1% SDS 30 min; 1 SSC 0,1% SDS 15 min, 0,2 SSC 0,1% SDS 15 min).

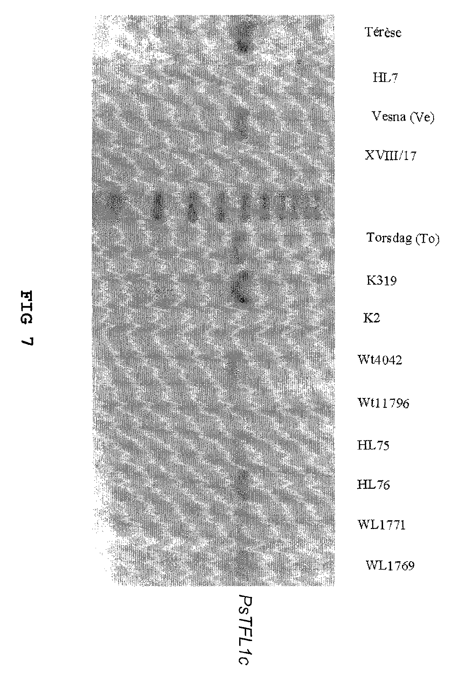

The results are shown in Figure 7.

Southern blot analysis reveals no detectable change for *If* or *Lf* lines or for *lf-a* line HL76.

In contrast, no band can be detected for the 4 *lf-a* lines HL7, XVIII/17, Wt11796 and K2. These results confirm that the *PsTFL1c* gene is not present in these *lf-a* lines. These lines were obtained from mutagenesis programs using Neutron or Gamma ray mutagenesis agents (Taylor, 1993) and therefore may represent deletion mutants.

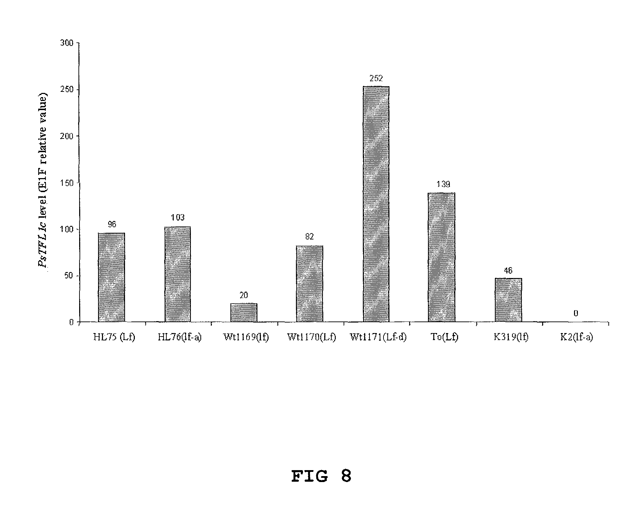

Level of *PsTFL1c* transcript was analysed by Real Time PCR on RNA extracted from the apex of plants from some of these lines at node 5 before floral initiation. In order to compare the results, the transcript level was also evaluated for the elongation factor, *E1F,* which is supposed to be constant in our conditions. *PsTFL1c* transcript level was estimated considering the level of the constitutive *E1F* gene. For each condition, the number of cycles necessary to reach a certain level of fluorescence was evaluated for *E1F* (n_{E1F}) and *PsTFL1c* (n_{PsTFL1c}). Difference between the two genes was calculated (d1= n_{PsTFL1c} - n_{E1F}). The value (2^{d1}) represents the difference of expression between *PsTFL1c* and the constitutive gene, *E1F.* This value was multiplied by 100000 as *E1F* is expressed at a higher level than *PsTFL1c.* The results are shown in Figure 8 and in Table I below.

**Table I**

| Lines | Progenitor Lines | Alleles at the *LF* locus | PsTFL1c amplification by PCR^{a} | Haplotype | *PsTFL1c* transcripts level^{b} |
|---|---|---|---|---|---|
| Térèse | WT | *Lf* | + | A | Nd |
| HL7 | Natural | *lf-a* | - | 0 | Nd |
| Vesna (Ve) | WT | *Lf-d* | + | A | Nd |
| XVIII/17 | Ve | *lf-a* | - | 0 | Nd |
| Torsdag (To) | WT | *Lf* | + | A | 139 |
| K319 | To | *lf* | + | C | 46 |
| K2 | To | *lf-a* | - | 0 | 0 |
| Wt4042 | WT | *Lf* | + | A | Nd |
| Wt11796 | Wt4042 | *lf-a* | - | 0 | Nd |
| HL75 | WT | *Lf* | + | A | 96 |
| HL76 | HL75 | *lf-a* | + | B | 103 |
| WL1771 | WT | *Lf-d* | + | D | 252 |
| WL1769 | WL1771 | *lf* | + | D | 46 |
| WL1770 | WL1771 | *Lf* | + | D | 139 |
| Porta (Po) | WT | *Lf* | + | A | Nd |
| Wt11790 | Po | *lf* | + | C | Nd |
| Wt11791 | Po | *lf* | + | C | Nd |
| Paloma (Pa) | WT | *Lf* | + | A | Nd |
| Wt11795 | Pa | *lf-a* | + | E | Nd |
| Champagne | WT | *Lf-d* | + | A | Nd |

| | | | | | |
|---|---|---|---|---|---|
| a "+": an amplicon was obtained; "-" : no amplification detected. | | | | | |
| b Non determined | | | | | |

A large variability in the level of transcripts was found. In the mutant series (WL1770 and WL1769), derived from the WL1771 progenitor lines, the expression of *PsTFL1c* is higher in WL1771 (*Lf-d*) than in WL1770 (*Lf*), and higher in WL1770 than in WL1769 (*lf*). Expression is, therefore, stronger in late alleles (*Lf-d*) than in early alleles (*lf*). Between *Lf-d* and *lf*, the difference is about 13 times.

Same results were found with the Torsdag series: To (*Lf*), K319 (*lf*) and K2 (*lf-a*). *PsTFL1c* is 3 times more expressed in late allele (*Lf*) than in early one (*lf*). No expression was detected in K2: this mutant has been showed to be a deletion mutant.

On the other hand, analysis of HL76 (*lf-a;* having a 6bp deletion in *PsTFL1c*) shows clearly that there is no association between the level of transcription of *PsTFL1c* in this mutant and early flowering. The *PsTFL1c* transcript level is the same between HL75 (*Lf*) and HL 76 (*lf-a*). The early flowering may be explained by the mutation in the PsTFL1c protein in HL76.

The above observations give strong evidence that changes of *PsTFL1c* transcript levels in *Lf* and *lf* mutants is due to change at the *LF* locus, and is not a consequence, but a cause of the early flowering phenotype.

*PsTFL1c* was mapped on linkage group II in the vicinity of the known gene, *Late Flowering.* To investigate this colocalization, *PsTFL1c* gene was sequenced in strong *Lf* mutants, *lf-a* (plants presenting an early flowering phenotype). 6 different *lf-a* mutants were analyzed. 4 were shown to be deletion mutants. No *PsTFL1c* genes can de detected in these lines either by PCR or by Southern blot. The two other *lf-a* lines present non-silent changes in the amino acid sequence. These changes are present in conserved amino acid (when compared with other plant *TFL1* genes) and therefore may result in non-functional protein that can account for the *lf-a* phenotype. These results give strong evidence that *PsTFL1c* corresponds to *Late flowering.*

Sequencing of the *Lf-d, Lf* and *lf* alleles revealed no change in the PsTFL1c amino acid sequence. These results indicated that the flowering phenotype of Lf mutants is not due to change of the protein (and therefore protein activity). The phenotype observed can be due to change of *PsTFL1c* transcription (difference in the spatial and/or temporal regulation of the gene) in the difference *Lf* lines. Analysis of *PsTFL1c* transcript level in apex before floral initiation shows a higher level of PsTFL1c transcript in the late flowering allele lines (*Lf-d*), than in the early ones (lf). Therefore difference of flowering time is due to change in PsTFL1c expression in *Lf* and *lf* lines. Such a regulation have already be described in tomato where the *fw2.2* alleles regulate the fruit size through changes in gene regulation, rather than the FW2.2 protein itself (Cong, 2002).

### EXAMPLE 5: THE PSTFL1 GENES IN PEA PRESENT DIFFERENT PATTERNS OF EXPRESSION.

By Northern blot, using *PsTFL1a* as a probe, a signal can be detected only in roots (data not shown). Due to possible cross-hybridization between the different *PsTf11* genes, it was difficult to say if the signal was due to *PsTFL1a, b* or *c*. So the expression of the *Tfl1* homologous genes was studied by RT-PCR in different tissues during the vegetative and reproductive phases under different daylenght conditions.

PCR was performed on cDNA, obtained from root, dormant node, internode, leaf, apex from vegetative plants, flower bud and flowers and from apex during the floral transition under short day or long day conditions.

Specific primers were designed for the different *PsTFL1* genes. Primers were chosen surrounding an intron to confirm if the signal observed is not due to genomic DNA contamination.

RNA were extracted using the RNA easy plant mini-kit (Qiagen, Hilden, Germany). 5 µg of total RNA is using for cDNA synthesis. Prior to reverse transcription, total RNA are treated with Amplification Grade Dnase I (Cat n° 18047-015, Life Technology) according to the manufacturer protocol. Reverse transcription is performed using 200 U of Superscript II Rnase H- reverse transcriptase (Cat n° 18064-022, Life Technology) in presence of 40 U of recombinant ribonuclease inhibitor (Cat n° 10777-019, Life Technology) with the AP primer (SEQ ID NO:17): 5'GGC CAC GCG TCG ACT AGT ACT TTT TTT TTT TTT TTT T 3'.

For PCR reaction, cDNA are diluted in 100 µl, and 1µl is using per reaction. RT-PCR is performed using specific primers:
- for *PsTFL1a* : 
(TFL5RACE (SEQ ID NO:18):
5' TGA GTT GTA CTC TTA AGT TCT TC 3';
 
TFLaR5 (SEQ ID NO:19):
5' AGG GCC AGG AAC ATC AGG GTC 3');
- for *PsTFL1b* : 
TFL1bR1(SEQ ID NO:14);
TFL1b31 (SEQ ID NO:20):
5' GAC AGA TAT ACC AGG CAC AAC AG 3')
 
TFL1bR1(SEQ ID NO:14);
TFL1b31 (SEQ ID NO:20):
5' GAC AGA TAT ACC AGG CAC AAC AG 3')
- for *PsTFL1c* : 
(TFLcR2 (SEQ ID NO:21):
5' AAA TAA GCA GCA GCA ACA GGG 3';
 
TFLcR3 (SEQ ID NO:22):
5' CAG ACA TTC CAG GGA CAA CAG 3';
and the following program (40 cycles at 94°C 30s, 60°C 60s and 72°C 30s).
The PCR reaction is loaded on ethidium bromide stained agarose gel.
As control the PCR was performed on genomic DNA and water.

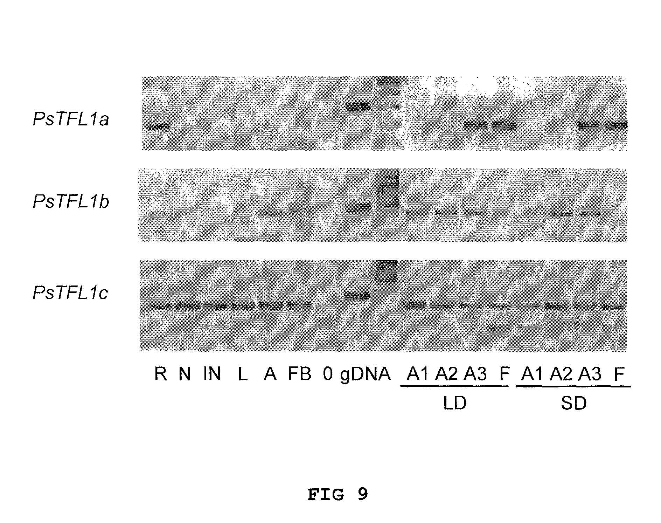

The results are shown in Figure 9.

*PsTFL1a* was expressed mainly in roots and the apex during floral development (flower buds and flowers). The expression in the apex was dependent on the stage of the plant. No expression of *PsTFL1a* was detected before the floral transition in SD or LD conditions. After the floral transition (stage A2), a signal was detected and remains in the apex after flowering (stage A3). No difference was observed between SD and LD.

This pattern of expression indicates a possible role of PsTFL1a in floral development.

For *PsTFL1b,* expression was found in the apex during the vegetative phase (Figure 9). Weaker expression was found in roots and dormant nodes. No expression was detected in the flower. *PsTFL1b* transcripts were detected in the apex before and after floral initiation. As for *PsTFL1a,* no difference was detected between SD and LD conditions. *PsTFL1b* expression is limited to vegetative organs.

Concerning *PsTFL1c* expression, no differences were observed. The *PsTFL1c* transcripts were present in all tissues studied (root, leaves, nodes, apex, flower, flower bud, (Figure 9)) and no difference in expression pattern during the floral process was detected. Therefore in our conditions, *PsTFL1c* was constitutively expressed during development.

### EXAMPLE 6: EXPRESSION OF PSTFL1A IN THE ROOT IS NOT SUFFICIENT TO MAINTAIN THE INDETERMINANCY OF THE APICAL MERISTEM.

The expression studies reveal that during the vegetative phase and during the floral transition, *PsTFL1a* is expressed in the root. In order to test whether this expression in the root has an effect on the growth of the apical meristem, graft experiments between wild type and *det1* plants were performed. Grafts (Scion/root stock) were grown in greenhouse conditions. For each combination, 10 plants were scored. The number of days from sowing to flowering, the node of flowering and the phenotype (wild type (WT) or determinate (det) were scored for each plant. The average and the standard deviation were calculated.

The results are shown in Table II below.

**Table II**

| Scion / Root stock | Phenotype observed | Number of days for flowering | | Flowering node | |
|---|---|---|---|---|---|
| | | Average | Standard deviation | Average | Standard deviation |
| WT/WT | WT | 45,6 | 1,9 | 18,8 | 1,1 |
| *det*/*det* | det | 45,9 | 1,8 | 18,3 | 0,8 |
| WT/*det* | WT | 46,7 | 3 | 17,7 | 1,3 |
| *det*/WT | det | 45,4 | 2,8 | 18,8 | 0,6 |

The *det* phenotype was only found in plants with a *det* genotype in the scion. When a *det* root was grafted on a wild type shoot, the plants have an indeterminate growth, whereas the opposite grafts (wild type as a root and *det* as a shoot) lead to determinate plants. The root genotype has no effect on the determinancy of the shoot, which depends only on the shoot genotype. So the *PsTFL1a* expression in the root has no influence on the shoot growth and floral development.

Moreover no significant differences in flowering nodes or in flowering time (determined when a flower is fully expended) was detected between wild type and *det* plants. The first flower develops in average at node 18 and opens after 46 days for almost all the plants in our conditions.

### REFERENCES:

* **Altschul, O. J., and Bradley, D. J.** (1997). Nucleic Acids Res *25*, 3389-3402.
* **Amaya, I., Ratcliffe, O. J., and Bradley, D. J.** (1999) Plant Cell *11,* 1405-18.
* **Banfield, M. J., and Brady, R. L.** (2000). J Mol Biol *297*, 1159-70.
* **Berdnikov, V. A., Gorel, F. L., Bogdanova, V. S., Kosterin, O. E., Trusov, Y. A., and Rpzov, S. M.** (1999). L. Genet. Res. *73*, 93-109.
* **Beveridge, C. A.** (2000). Plant Growth Regulation *32*, 193-203.
* **Blasquez, M. A.** (2000). Journal of cell science *113,* 3547-3548.
* **Bradley, D., Carpenter, R., Copsey, L., Vincent, C.**, **Rothstein, S., and Coen, E.** (1996). Nature *379*, 791-7.
* **Bradley, D., Ratcliffe, O., Vincent, C., Carpenter, R., and Coen, E.** (1997). Science *275*, 80-3.
* **Church, G. M., and Guilbert, W.** (1984). Proc. Natl. Acad. Sci. USA *81,* 1991-1995.
* **Coen, E. S., Romero, J. M., Doyle, S., Elliott, R., Murphy, G., and Carpenter, R.** (1990). Cell *63*, 1311-22.
* **Colasanti, J., and Sundaresan, V.** (2000). Trends Biochem Sci *25*, 236-40.
* **Cong, O. J., and Bradley, D. J.** (2002). Plant Cell *11,* 1405-18.
* **Corpet, F.** (1988). Nucleic Acids Res *16*, 10881-90.
* **Ellis, T. H., Turner, L., Hellens, R. P., Lee, D., Harker, C. L., Enard, C., Domoney, C., and Davies, D. R.** (1992). Genetics *130*, 649-63.
* **Hofer, J., Turner, L., Hellens, R., Ambrose, M., Matthews, P., Michael, A., and Ellis, N.** (1997). Curr Biol 7, 581-7.
* **Jensen, C. S., Salchert, K., and Nielsen, K. K.** (2001). Plant Physiol *125*, 1517-28.
* **Kardailsky, I., Shukla, V. K., Ahn, J. H., Dagenais, N., Christensen, S. K., Nguyen, J. T., Chory, J., Harrison, M. J., and Weigel, D.** (1999). Science *286*, 1962-5.
* **Kobayashi, Y., Kaya, H., Goto, K., Iwabuchi, M., and Araki, T.** (1999). Science *286*, 1960-2.
* **Laucou, V., Haurogné, K., Ellis, N., and Rameau, C.** (1998). Theor Appl Genet *97*, 905-915.
* **Michaels, S. D., and Amasino, R. M.** (1998). Plant J *14*, 381-5.
* **Mimida, N., Goto, K., Kobayashi, Y., Araki, T., Ahn, J. H., Weigel, D., Murata, M., Motoyoshi, F., and Sakamoto, W.** (2001). Genes Cells *6*, 327-36.
* **Murfet, I. C.** (1971). Aust J Biol Sci *24*, 1089-1101.
* **Murfet, I. C.** (1989). PNL *21*, 44-47.
* **Neff, M. M., Neff, J. D., Chory, J., and Pepper, A. E.** (1998). Plant J *14*, 387-92.
* **Ohshima, S., Murata, M., Sakamoto, W., Ogura, Y., and Motoyoshi, F.** (1997). Mol Gen Genet *254*, 186-94.
* **Pnueli, L., Carmel-Goren, L., Hareven, D., Gutfinger, T., Alvarez, J., Ganal, M., Zamir, D., and Lifschitz, E.** (1998). Development *125*, 1979-89.
* **Pnueli, L., Gutfinger, T., Hareven, D., Ben-Naim, O., Ron, N., Adir, N., and Lifschitz, E.** (2001). Plant Cell *13*, 2687-702.
* **Ratcliffe, O. J., Amaya, I., Vincent, C. A., Rothstein, S., Carpenter, R., Coen, E. S., and Bradley, D. J.** (1998). Development *125*, 1609-15.
* **Reid, J. B., and Murfet, I. C.** (1984). Ann. Bot. *53*, 369-382.
* **Reid, J. B., Murfet, I. C., Singer, S. R., Weller, J. L., and Taylor, S. A.** (1996). Cell and Developmental Biology 7, 455-463.
* **Sambrook, J., Fritsch, E. F., and Maniatis, T.** (1989). Molecular cloning: a laboratory manual (New York: Cold Spring Harbor Laboratory Press).
* **Singer, S., Sollinger, J., Maki, S., Fishbach, J., Short, B., Reinke, J., Fick, J., Cox, L., McCall, A., and Mullen, H.** (1999). The botanical review *65*, 385-410.
* **Singer, S. R., Hsuing, L. P., and Huber, S. C.** (1991). Am J. Bot. *77*, 1330-1335.
* **Souer, E., van der Krol, A., Kloos, D., Spelt, C., Bliek, M., Mol, J., and Koes, R.** (1998). Development *125*, 733-42.
* **Swiecicki, W. K.** (1987). PNL *19*, 72.
* **Taylor, O. J., and Bradley, D. J.** (1993). Plant Cell *11*, 1405-18.
* **Thompson, J. D., Higgins, D. G., and Gibson, T. J.** (1994). Nucleic Acids Res *22*, 4673-80.
* **Weigel, D., Alvarez, J., Smyth, D. R., Yanofsky, M. F., and Meyerowitz, E. M.** (1992). Cell *69*, 843-59.
* **Weller, J. L., Reid, J. B., Taylor, S. A., and Murfet, I. C.** (1977). Trends in plant science *2*, 412-418.

## Claims

1. An isolated polypeptide active in floral transition and/or floral development, wherein said polypeptide is selected among:
- a polypeptide hereinafter referred as PsTFL1c, having at least 75% identity with the polypeptide SEQ ID NO :4;
- a polypeptide hereinafter referred as PsTFL1a, having at least 75% identity with the polypeptide SEQ ID NO :2.

2. A variant of a polypeptide of claim 1, wherein said variant is selected among:
- a variant of PsTFL1c polypeptide wherein the phenylalanine, alanine, and aspartic acid residues at positions 147-149 of said polypeptide are replaced by a tyrosine;
- a variant of PsTFL1c polypeptide wherein the proline residue at position 111 of said polypeptide is replaced by a serine;
- a variant of PsTFL1a polypeptide consisting of the 106 C-terminal amino acids thereof;
- a variant of PsTFL1a polypeptide resulting from the replacement of the glutamine residue at position 127 of said polypeptide by an arginine.
- a variant of PsTFL1a polypeptide resulting from the replacement of the glutamic acid residue at position 104 of said polypeptide by a lysine.

3. An isolated polypeptide selected among:
- a fragment of at least 10 consecutive aminoacid residues of a polypeptide of any of claims 1 or 2.
- a chimeric polypeptide comprising a polypeptide of any of claims 1 or 2 or a fragment of at least 10 consecutive aminoacid residues thereof.

4. An isolated polynucleotide selected among:
a) a polynucleotide encoding a polypeptide of any of claims 1 to 3, and its complement;
b) a polynucleotide which hybridizes under high stringency conditions with a polynucleotide a), and its complement;
c) a fragment of at least 10 consecutive nucleotides of a polynucleotide a) or b) above;
d) the complement of a polynucleotide a), b), or c) above.

5. An isolated polynucleotide of claim 4, wherein polynucleotide a) is selected among:
- a polynucleotide comprising a nucleotide sequence having at least 75% identity with SEQ ID NO : 3;
- a polynucleotide comprising a nucleotide sequence having at least 75% identity with SEQ ID NO : 1.

6. A nucleic acid probe containing at least a fragment of at least 10 consecutive nucleotides of a polynucleotide of any of claims 4 or 5.

7. A pair of primers comprising at least a primer consisting of a fragment of at least 10 consecutive nucleotides of a polynucleotide of any of claims 4 or 5.

8. A DNA construct comprising a polynucleotide of any of claims 4 or 5.

9. A host cell transformed by a polynucleotide of any of claims 4 or 5

10. A host cell according to claim 9, wherein said cell is a plant cell.

11. A transgenic plant genetically modified by a polynucleotide of any of claims 4 or 5.

12. A transgenic plant of claim 11, selected among:
- transgenic plants wherein said polynucleotide expresses a polypeptide of claim 1;
- transgenic plants wherein said polynucleotide expresses a polypeptide variant of claim 2;
- transgenic plants wherein said polynucleotide induces the silencing of a gene encoding a polypeptide of claim 1.

13. A method for regulating the floral transition and/or floral development in a plant wherein said method comprises modulating the expression of at least one polypeptide of claim 1 in said plant.

14. A method for identifying an allele, associated with a given flowering phenotype, of a gene encoding a polypeptide of claim 1 wherein said method comprises isolating a nucleic acid fragment comprising said gene or a portion thereof from at least one plant expressing said phenotype, and sequencing said fragment.

15. A method for identifying polymorphisms associated with flowering phenotype, in a gene encoding a polypeptide of claim 1 wherein said method comprises identifying by the method of claim 14, at least two alleles of said gene associated with different flowering phenotypes and comparing the sequences of said alleles.

16. A method for testing a plant for its flowering properties wherein said method comprises detecting whether an allele associated with a given flowering phenotype of a gene encoding a polypeptide of claim 1 is present in said plant.

17. A method of claim 16, wherein the allele to be detected encodes a polypeptide of claim 2.

18. A transgenic plant of any of claims 11 or 12, wherein said plant is a Fabaceae.

19. A method of any of claims 13 to 17, wherein said plant is a Fabaceae.


SEQUENCE LISTING

<110>  GENOPLANTE VALOR

<120>  Polypeptides involved in floral initiation and genes encoding the same.

<130>  MJPbv1516/12

<160>  22

<170>  PatentIn version 3.1

<210>  1

<211>  730

<212>  DNA

<213>  Pisum sativum

<220>

<221>  CDS

<222>  (1)..(522)

<223>

<400>  1
atg gca aga atg gct caa gaa cca ctc atc gtt ggt aga gtc ata ggt        48
Met Ala Arg Met Ala Gln Glu Pro Leu Ile Val Gly Arg Val Ile Gly
1               5                   10                  15

gaa gtt ctt gat tcc ttt acc aca agc atg aaa atg act gtt agt tac        96
Glu Val Leu Asp Ser Phe Thr Thr Ser Met Lys Met Thr Val Ser Tyr
            20                  25                  30

aac aag aaa caa gtc ttc aat ggc cat gag ttt ttc cct tcc acc atc       144
Asn Lys Lys Gln Val Phe Asn Gly His Glu Phe Phe Pro Ser Thr Ile
        35                  40                  45

aac acc aaa ccc aaa gtt gag att gat ggc gct gac atg aga tcc ttc       192
Asn Thr Lys Pro Lys Val Glu Ile Asp Gly Ala Asp Met Arg Ser Phe
    50                  55                  60

tat aca ctg gtg atg aca gac cct gat gtt cct ggc cct agt gat cct     240
Tyr Thr Leu Val Met Thr Asp Pro Asp Val Pro Gly Pro Ser Asp Pro
65                  70                  75                  80

tat cta aga gaa cac ttg cac tgg att gtg aca gat att cca gga aca     288
Tyr Leu Arg Glu His Leu His Trp Ile Val Thr Asp Ile Pro Gly Thr
                85                  90                  95

aca gat gct aca ttt ggg aaa gag ata gta agc tat gaa att cca aag     336
Thr Asp Ala Thr Phe Gly Lys Glu Ile Val Ser Tyr Glu Ile Pro Lys
            100                 105                 110

cca aat ata gga ata cac agg ttt gtg ttt gtt cta ttc aaa caa aga     384
Pro Asn Ile Gly Ile His Arg Phe Val Phe Val Leu Phe Lys Gln Arg
        115                 120                 125

gcg aga gat tca gtg aga gca aca ccg tct tca aga gac cat ttc aac     432
Ala Arg Asp Ser Val Arg Ala Thr Pro Ser Ser Arg Asp His Phe Asn

    130                 135                 140

act cgt agc ttt gct tca cag aac gat ctt ggt ctt cct gtt gct gct     480
Thr Arg Ser Phe Ala Ser Gln Asn Asp Leu Gly Leu Pro Val Ala Ala
145                 150                 155                 160

gtt tac ttc aat gct cag aga gaa acc gct gca aga aga cgc             522
Val Tyr Phe Asn Ala Gln Arg Glu Thr Ala Ala Arg Arg Arg
                165                 170

tagtatacat gctataaacc tagtttggtc tgtttttgac gtacttgtgt tgcagaaagg     582

gaatcatttt ctgtgtgttg ttagttactg tccattggat atatatatta ttgtactgta     642

tgtttgttat gtgtcgtgtg tgtatcaaag tagtaatctt ttatcttgtt atattataca     702

tttcaagaaa taagagaggt tgttatat                                        730


<210>  2

<211>  174

<212>  PRT

<213>  Pisum sativum


<220>

<221>  VARIANT

<222>  (4)..(4)

<223>  det 2 et det 3
       M replaced by I

<220>

<221> VARIANT

<222> (47)..(47)

<223> det 2
      T replaced by P

<220>

<221> VARIANT

<222> (66)..(66)

<223> det 3
      T replaced by I

<220>

<221> VARIANT

<222> (1)..(68)

<223> det 1
      deletion

<220>

<221> VARIANT

<222> (104)..(104)

<223> det 3
      E replaced by K

<220>

<221> VARIANT

<222> (105)..(105)

<223> det 1
      I replaced by V

<220>

<221> VARIANT

<222> (110)..(110)

<223>  det 1
      I replaced by T

<220>

<221>  VARIANT

<222>  (127)..(127)

<223>  det 2
      Q replaced by R

<400>  2

Met Ala Arg Met Ala Gln Glu Pro Leu Ile Val Gly Arg Val Ile Gly
1               5                   10                  15

Glu Val Leu Asp Ser Phe Thr Thr Ser Met Lys Met Thr Val Ser Tyr
            20                  25                  30

Asn Lys Lys Gln Val Phe Asn Gly His Glu Phe Phe Pro Ser Thr Ile
        35                  40                  45

Asn Thr Lys Pro Lys Val Glu Ile Asp Gly Ala Asp Met Arg Ser Phe
    50                  55                  60

Tyr Thr Leu Val Met Thr Asp Pro Asp Val Pro Gly Pro Ser Asp Pro
65                  70                  75                  80

Tyr Leu Arg Glu His Leu His Trp Ile Val Thr Asp Ile Pro Gly Thr
                85                  90                  95

Thr Asp Ala Thr Phe Gly Lys Glu Ile Val Ser Tyr Glu Ile Pro Lys
            100                 105                 110

Pro Asn Ile Gly Ile His Arg Phe Val Phe Val Leu Phe Lys Gln Arg
        115                 120                 125

Ala Arg Asp Ser Val Arg Ala Thr Pro Ser Ser Arg Asp His Phe Asn
    130                 135                 140

Thr Arg Ser Phe Ala Ser Gln Asn Asp Leu Gly Leu Pro Val Ala Ala
145                 150                 155                 160

Val Tyr Phe Asn Ala Gln Arg Glu Thr Ala Ala Arg Arg Arg
                165                 170

<210>  3

<211>  784

<212>  DNA

<213>  Pisum sativum



<220>

<221>  CDS

<222>  (1)..(519)

<223>



<400>  3

atg aat tca gat cct cta att ctt ggg aga gtg ata gga gat gta att        48
Met Asn Ser Asp Pro Leu Ile Leu Gly Arg Val Ile Gly Asp Val Ile
1               5                   10                  15

gat tat ttt acc gca agc ata aaa atg tct gta att tac aac aac aaa        96
Asp Tyr Phe Thr Ala Ser Ile Lys Met Ser Val Ile Tyr Asn Asn Lys
            20                  25                  30

gaa atc ttt act gga tat gaa gta ccc ttt cct tct aca gtt aag act       144
Glu Ile Phe Thr Gly Tyr Glu Val Pro Phe Pro Ser Thr Val Lys Thr
        35                  40                  45

aag cca agg att cag att caa gga ggg gac atg agg tcc ctc ttt acc       192
Lys Pro Arg Ile Gln Ile Gln Gly Gly Asp Met Arg Ser Leu Phe Thr
    50                  55                  60

ctg atc atg ata gac cca gat gtt cct ggc cca agt gat cct tac atg       240
Leu Ile Met Ile Asp Pro Asp Val Pro Gly Pro Ser Asp Pro Tyr Met
65                  70                  75                  80

aaa gaa cac ttg cgc tgg atg gtg aca gac att cca ggg aca aca gat       288
Lys Glu His Leu Arg Trp Met Val Thr Asp Ile Pro Gly Thr Thr Asp
                85                  90                  95

tcc aca ttt gga aaa gag ttg aca agc tat gag aaa cca aag cct aat       336
Ser Thr Phe Gly Lys Glu Leu Thr Ser Tyr Glu Lys Pro Lys Pro Asn
            100                 105                 110

ata gga atc cat aga tat gtg ttt gtc ctt ttc aag caa aaa agg ggg       384
Ile Gly Ile His Arg Tyr Val Phe Val Leu Phe Lys Gln Lys Arg Gly
        115                 120                 125

aac aag tac tcc att aca tgt cct ttt tcg agg gat cac ttc aac aca       432
Asn Lys Tyr Ser Ile Thr Cys Pro Phe Ser Arg Asp His Phe Asn Thr
    130                 135                 140

cga aat ttt gca gat caa aat gac ctt ggt gtc cct gtt gct gct gct       480
Arg Asn Phe Ala Asp Gln Asn Asp Leu Gly Val Pro Val Ala Ala Ala

145                 150                 155                 160

tat ttc aat gct aga agg gca acg gct cct aga aga cgc taggggttag      529
Tyr Phe Asn Ala Arg Arg Ala Thr Ala Pro Arg Arg Arg
                165                 170

gctactatag ccatccaatc agaagccgat tatcatgact ttatggctat gattgaaata      589

acagctttag cttgctttag attgatttag ctttcttgtg tttgtggagt tgttaagaac      649

aaagatgtta gaagttaatg aaattcagaa gcatggatat tgttgtgaat gccatgctag      709

ctgattgatg tttgtttgtt tagaaattat tgtagctttt ggtgtggtaa tgctcaattt      769

ctttctagtt aaaaa                                                      784

<210>  4

<211>  173

<212>  PRT

<213>  Pisum sativum

<220>

<221>  VARIANT

<222>  (111)..(111)

<223>  HL 76
       P replaced by S

<220>

<221>  VARIANT

<222>  (147)..(149)

<223>  Wt11795
       FAD replaced by Y

<400>  4

Met Asn Ser Asp Pro Leu Ile Leu Gly Arg Val Ile Gly Asp Val Ile
1               5                   10                  15

Asp Tyr Phe Thr Ala Ser Ile Lys Met Ser Val Ile Tyr Asn Asn Lys
            20                  25                  30

Glu Ile Phe Thr Gly Tyr Glu Val Pro Phe Pro Ser Thr Val Lys Thr
        35                  40                  45

Lys Pro Arg Ile Gln Ile Gln Gly Gly Asp Met Arg Ser Leu Phe Thr
    50                  55                  60

Leu Ile Met Ile Asp Pro Asp Val Pro Gly Pro Ser Asp Pro Tyr Met
65                  70                  75                  80

Lys Glu His Leu His Trp Met Val Thr Asp Ile Pro Gly Thr Thr Asp
                85                  90                  95

Ser Thr Phe Gly Lys Glu Leu Thr Ser Tyr Glu Lys Pro Lys Pro Asn
            100                 105                 110

Ile Gly Ile His Arg Tyr Val Phe Val Leu Phe Lys Gln Lys Arg Gly
        115                 120                 125

Asn Lys Tyr Ser Ile Thr Cys Pro Phe Ser Arg Asp His Phe Asn Thr
    130                 135                 140

Arg Asn Phe Ala Asp Gln Asn Asp Leu Gly Val Pro Val Ala Ala Ala
145                 150                 155                 160

Tyr Phe Asn Ala Arg Arg Ala Thr Ala Pro Arg Arg Arg
                165                 170

<210>  5

<211>  1114

<212>  DNA

<213>  Pisum sativum

<220>

<221>  5'UTR

<222>  (1)..(16)

<223>

<220>

<221>  exon

<222>  (17)..(217)

<223>

<220>

<221>  Intron

<222>  (218)..(339)

<223>

<220>

<221>  exon

<222>  (340)..(402)

<223>

<220>

<221>  Intron

<222>  (403)..(474)

<223>

<220>

<221>  exon

<222>  (475)..(513)

<223>

<220>

<221>  Intron

<222>  (514)..(594)

<223>

<220>

<221>  exon

<222>  (595)..(816)

<223>

<220>

<221>  3'UTR

<222>  (817)..(1114)

<223>

<400>  5

ttcactcttt tctaaa atg gca aga atg gct caa gaa cca ctc atc gtt ggt      52
                  Met Ala Arg Met Ala Gln Glu Pro Leu Ile Val Gly
                  1               5                   10

aga gtc ata ggt gaa gtt ctt gat tcc ttt acc aca agc atg aaa atg      100
Arg Val Ile Gly Glu Val Leu Asp Ser Phe Thr Thr Ser Met Lys Met
        15                  20                  25

act gtt agt tac aac aag aaa caa gtc ttc aat ggc cat gag ttt ttc      148
Thr Val Ser Tyr Asn Lys Lys Gln Val Phe Asn Gly His Glu Phe Phe
    30                  35                  40

cct tcc acc atc aac acc aaa ccc aaa gtt gag att gat ggc gct gac      196
Pro Ser Thr Ile Asn Thr Lys Pro Lys Val Glu Ile Asp Gly Ala Asp
45                  50                  55                  60

atg aga tcc ttc tat aca ctg gtaattctct ttatttaaga aaaacaaaca      247
Met Arg Ser Phe Tyr Thr Leu
                65

tgaacagtta attcaatgtt tttgttgttt tctatattct tgtatatttg acctaatatg      307

tttatataat tgtcttccat ttatgtctct ag gtg atg aca gac cct gat gtt      360
                                    Val Met Thr Asp Pro Asp Val
                                            70

cct ggc cct agt gat cct tat cta aga gaa cac ttg cac tgg      402
Pro Gly Pro Ser Asp Pro Tyr Leu Arg Glu His Leu His Trp
75                  80                  85

taaatataat ataaccatat tatagctaga aattaatttt ctttgattaa atttttgttt      462

actcattgca gg att gtg aca gat att cca gga aca aca gat gct aca ttt      513
              Ile Val Thr Asp Ile Pro Gly Thr Thr Asp Ala Thr Phe
                  90                  95                  100

ggtaaatatt tttattggtt aaaaaatgta tatatatgga tgatgagaga gaatgacata      573

ttgaatgatt ttttttttat a ggg aaa gag ata gta agc tat gaa att cca      624
                        Gly Lys Glu Ile Val Ser Tyr Glu Ile Pro
                                    105                 110

aag cca aat ata gga ata cac agg ttt gtg ttt gtt cta ttc aaa caa      672

Lys Pro Asn Ile Gly Ile His Arg Phe Val Phe Val Leu Phe Lys Gln
            115                 120                 125

aga gcg aga gat tca gtg aga gca aca ccg tct tca aga gac cat ttc      720
Arg Ala Arg Asp Ser Val Arg Ala Thr Pro Ser Ser Arg Asp His Phe
        130                 135                 140

aac act cgt agc ttt gct tca cag aac gat ctt ggt ctt cct gtt gct      768
Asn Thr Arg Ser Phe Ala Ser Gln Asn Asp Leu Gly Leu Pro Val Ala
    145                 150                 155

gct gtt tac ttc aat gct cag aga gaa acc gct gca aga aga cgc tag      816
Ala Val Tyr Phe Asn Ala Gln Arg Glu Thr Ala Ala Arg Arg Arg
160                 165                 170

ttttagctac tagctatgaa tgactatgat taattaagca aggttaatta attataataa    876

ataaagtgat taatagtaat atatatagta tagtatacat gctataaacc tagtttggtc    936

tgtttttgac gtacttgtgt tgcagaaagg gaatcatttt ctgtgtgttg ttagttactg    996

tccattggat atatatatta ttgtactgta tgtttgttat gtgtcgtgtg tgtatcaaag   1056

tagtaatctt ttatcttgtt atattataca tttcaagaaa taagagaggt tgttatat     1114

<210>  6

<211>  2267

<212>  DNA

<213>  Pisum sativum

<220>

<221>  5'UTR

<222>  (1)..(183)

<223>

<220>

<221>  exon

<222>  (184)..(378)

<223>

<220>

<221>  Intron

<222>  (379)..(537)

<223>

<220>

<221>  exon

<222>  (538)..(599)

<223>

<220>

<221>  Intron

<222>  (600)..(1640)

<223>

<220>

<221>  exon

<222>  (1641)..(1681)

<223>

<220>

<221>  Intron

<222>  (1682)..(1781)

<223>

<220>

<221>  exon

<222>  (1782)..(2005)

<223>

<220>

<221>  3'UTR

<222>  (2006)..(2267)

<223>

<400>  6

aaataaccga ataaataatt aaatggtaac ttcacctcta atatttttta atttttcaaa      60

ataacaccgt atagctctcg ttttaatgcc tctcagtata aataccatgc taactatagt     120

tgttattcat tcattcgtaa gcacttagta gttttattgt acttgttcat tctcttggga     180

agt atg aat tca gat cct cta att ctt ggg aga gtg ata gga gat gta       228
    Met Asn Ser Asp Pro Leu Ile Leu Gly Arg Val Ile Gly Asp Val
    1               5                   10                  15

att gat tat ttt acc gca agc ata aaa atg tct gta att tac aac aac       276
Ile Asp Tyr Phe Thr Ala Ser Ile Lys Met Ser Val Ile Tyr Asn Asn
                20                  25                  30

aaa gaa atc ttt act gga tat gaa gta ccc ttt cct tct aca gtt aag       324
Lys Glu Ile Phe Thr Gly Tyr Glu Val Pro Phe Pro Ser Thr Val Lys
            35                  40                  45

act aag cca agg att cag att caa gga ggg gac atg agg tcc ctc ttt       372
Thr Lys Pro Arg Ile Gln Ile Gln Gly Gly Asp Met Arg Ser Leu Phe
        50                  55                  60

acc ctg gtattaacta tatatatttc ttatactttt tttatttgtg tttagacatc        428
Thr Leu
    65

ttaagtgatt aatagatggt gtaaaaagct cttttttaag tgattattag atggtgtaaa     488

aagctctttt attagaaaaa taagttaatt atttattgtt ttcctgcag atc atg ata     546
                                                      Ile Met Ile

gac cca gat gtt cct ggc cca agt gat cct tac atg aaa gaa cac ttg       594
Asp Pro Asp Val Pro Gly Pro Ser Asp Pro Tyr Met Lys Glu His Leu
    70                  75                  80

cgc tg  gtatttaata tatcactatc ttgatcatat accttgaaat aggttatttt        649
Arg Trp
85

gtctctaaat cgattatata ccttgaaata taggttattt tgcctctaaa tcgatcgatc     709

tggttataaa tatttcaaca aagaaagttt aatatgtcag caaatcttag tttaactgta     769

gttaatattg gtttattatt aggaaccata gatttcacat ttatgtttct gagtttctag     829

tagtgagtta ctagtagtta ttactctttt cttccaataa aaaaagaaaa cttttaatgg     889

taataatcat ttaaagtggt aggaatactt atattcctaa aaaaatagaa ataaaactga     949

gtcaaaaatg aaaaattaaa taaaactatt tactataaaa catgcacaat aatagttact    1009

atatggaaga gtttgatcat ttgattgatc atttgatgct ttgatatatc aatatggaaa 1069

acttatgagg attagattag agacggtttg ttgtagtttt taaaaaatag ctttattttt 1129

ttatttttaa aaataaattt tataaaaata ttttttaaaa tattaatatt ttttaaattt 1189

attttttata aataacatta atttttatat catataacat agatatataa tattagagaa 1249

taaaatataa tcaaaaatat aattttaaaa ataatgtatc ttataaatat tttttatgaa 1309

aatttatttt aaatagcttc agatttaagt gtttttattt taactttggt ataaatatat 1369

taataataaa atgataaaat atttaaaata ttatttcaaa aatattcgaa ctgatttttc 1429

attagaattt ttttatatga gctaggttta agcgatttca tagccatagc ctataagtag 1489

atttttaaca tctaaagttt atttaccact taaggttgat tgattatggt gtaataatat 1549

taatttgcac attaggtgag taatgaaagt acattgtatt gaaagaaatg aatatccaat 1609

tttggttata actaataatg aatttttgta g g atg gtg aca gac att cca ggg 1662
Met Val Thr Asp Ile Pro Gly
            90

aca aca gat tcc aca ttt g gtaggttttg aacatagatt tattgaacat 1711
Thr Thr Asp Ser Thr Phe
    95

attaatgatg tttttgttga aagaacgtaa aagtttaccc taaattttct ccaacttggt 1771

gaatgtacag ga aaa gag ttg aca agc tat gag aaa cca aag cct aat 1819
Gly Lys Glu Leu Thr Ser Tyr Glu Lys Pro Lys Pro Asn
105 110

ata gga atc cat aga tat gtg ttt gtc ctt ttc aag caa aaa agg ggg 1867
Ile Gly Ile His Arg Tyr Val Phe Val Leu Phe Lys Gln Lys Arg Gly
        115                 120                 125

aac aag tac tcc att aca tgt cct ttt tcg agg gat cac ttc aac aca 1915
Asn Lys Tyr Ser Ile Thr Cys Pro Phe Ser Arg Asp His Phe Asn Thr
    130                 135                 140

cga aat ttt gca gat caa aat gac ctt ggt gtc cct gtt gct gct gct 1963
Arg Asn Phe Ala Asp Gln Asn Asp Leu Gly Val Pro Val Ala Ala Ala
145                 150                 155                 160

tat ttc aat gct aga agg gca acg gct cct aga aga cgc tag 2005
Tyr Phe Asn Ala Arg Arg Ala Thr Ala Pro Arg Arg Arg
                165                 170

gggttaggct actatagcca tccaatcaga agccgattat catgacttta tggctatgat 2065

tgaaataaca gctttagctt gctttagatt gatttagctt tcttgtgttt gtggagttgt 2125

taagaacaaa gatgttagaa gttaatgaaa ttcagaagca tggatattgt tgtgaatgcc 2185

atgctagctg attgatgttt gtttgtttag aaattattgt agcttttggt gtggtaatgc 2245

tcaatttctt tctagttaaa aa 2267

<210> 7

<211> 22

<212> DNA

<213> Artificial sequence

<220>

<223> DNA primer

<400> 7
atggggagag tgathggrga wg 22

<210> 8

<211> 23

<212> DNA

<213> artificial sequence

<220>

<223> DNA primer

<400> 8
tcactaggrc cwggaacatc wgg 23

<210> 9

<211> 23

<212> DNA

<213> artificial sequence

<220>

<223> DNA primer

<400> 9
gatgttccwg gwcctagtga ycc 23

<210> 10

<211> 20

<212> DNA

<213>  artificial sequence

<220>

<223>  DNA primer

<400>  10
cttgcagcrg tytcyctytg                                                    20

<210>  11

<211>  28

<212>  DNA

<213>  artificial sequence

<220>

<223>  DNA primer

<400>  11
gaacacttgc actggtaaat ataataga                                           28

<210>  12

<211>  21

<212>  DNA

<213>  artificial sequence

<220>

<223>  DNA primer

<400>  12
tgtagcatct gttgttcctg g                                                  21

<210>  13

<211>  21

<212>  DNA

<213>  artificial sequence

<220>

<223>  DNA primer

<400>  13
gaaatatgaa atgccacgtc c                                                21

<210>  14

<211>  23

<212>  DNA

<213>  artificial sequence

<220>

<223>  DNA primer

<400>  14
acaaactaga acaacaacaa ccc                                              23

<210>  15

<211>  22

<212>  DNA

<213>  artificial sequence

<220>

<223>  DNA primer

<400>  15
ctcgttttaa tgcctctcag ta                                               22

<210>  16

<211>  21

<212>  DNA

<213>  artificial sequence

<220>

<223>  DNA primer

<400>  16
gagcattacc acaccaaaag c                                                21

<210>  17

<211>  37

<212>  DNA

<213>  artificial sequence

<220>

<223>  DNA primer

<400>  17
ggccacgcgt cgactagtac tttttttttt ttttttt                                37

<210>  18

<211>  23

<212>  DNA

<213>  artificial sequence

<220>

<223>  DNA primer

<400>  18
tgagttgtac tcttaagttc ttc                                               23

<210>  19

<211>  21

<212>  DNA

<213>  artificial sequence

<220>

<223>  DNA primer

<400>  19
agggccagga acatcagggt c                                                 21

<210>  20

<211>  23

<212>  DNA

<213>  artificial sequence

<220>

<223>  DNA primer

<400>  20
gacagatata ccaggcacaa cag                                              23

<210>  21

<211>  21

<212>  DNA

<213>  artificial sequence

<220>

<223>  DNA primer

<400>  21
aaataagcag cagcaacagg g                                                21

<210>  22

<211>  21

<212>  DNA

<213>  artificial sequence

<220>

<223>  DNA primer

<400>  22
cagacattcc agggacaaca g                                                21